# EUROPEAN PATENT APPLICATION

(11) **EP 2 269 609 A2**
(43) Date of publication of application: **05.01.2011**
(21) Application number: 10183856.3
(22) Date of filing: 16.10.2002
(51) Int. Cl.: A61K 31/47, A61K 31/16, A61P 25/28, A61P 35/00

(54) **Treatment of neurodegenerative diseases and cancer of the brain with SAHA**

(30) Priority: 16.10.2001 US 329705 P
(62) Divisional of application: 02778601.1
(71) Applicant: Sloan-Kettering Institute for Cancer Research, New York, NY 10065 (US)
(72) Inventor: Richon, Victoria, M., Wellesley, MA 02481 (US); Marks, Paul, A., Washington, CT 06793 (US); Rifkind, Richard, A., New York, NY 10022 (US)
(74) Representative: Griffin, Philippa Jane

(57) **Abstract**

A composition comprising suberoylanilide hydroxamic acid (SAHA) represented by the following structure: 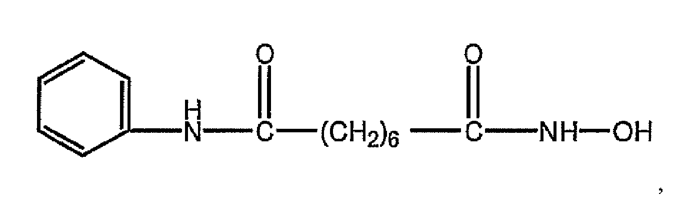 or a pharmaceutically acceptable salt or hydrate thereof, and a Pharmaceutical acceptable carrier or diluent, for use in the treatment of brain cancer in a mammal, wherein the composition is formulated for oral administration.

## Description

### RELATED APPLICATIONS

This application claims the benefit of U.S. Provisional Application No. 60/329,705 filed on October 16, 2001. The entire teachings of the above-referenced application are incorporated herein by reference.

### BACKGROUND OF THE INVENTION

Compounds which inhibit histone deacetylases (HDACs) have been shown to cause growth arrest, differentiation and/or apoptosis of many different types of tumor cells *in vitro* and *in vivo.* HDACs catalyze the removal of the acetyl group from the lysine residues in the N-terminal tails of nucleosomal core histones resulting in a more compact chromatin structure, a configuration that is generally associated with repression of transcription. These HDAC inhibitors fall into four general classes: 1) short-chain fatty acids (*e.g*., 4-phenylbutyrate and valproic acid); hydroxamic acids (*e*.*g*., SAHA, Pyroxamide, CBHA, trichostatin A (TSA), oxamflatin and CHAPs, such as, CHAP1 and CHAP 31); 3) cyclic tetrapeptides (Trapoxin A and Apicidin; 4) benzamides (*e*.*g*., MS-275); and other compounds such as Scriptaid. Examples of such compounds can be found in U.S. Patent Nos. 5,369,108, issued on November 29, 1994, 5,700,811, issued on December 23, 1997, and 5,773,474, issued on June 30, 1998 to Breslow et al., U.S. Patent Nos. 5,055,608, issued on October 8, 1991, and 5,175,191, issued on December 29, 1992 to Marks et al., as well as, Yoshida, M., et al., Bioassays 17, 423-430 (1995), Saito, A., et al., PNAS USA 96, 4592-4597, (1999), Furamai R. et al., PNAS USA 98 (1), 87-92 (2001), Komatsu, Y., et al., Cancer Res. 61(11), 4459-4466 (2001), Su, G.H., et al., Cancer Res. 60, 3137-3142 (2000), Lee, B.I. et al., Cancer Res. 61(3), 931-934, Suzuki, T., et al., J. Med. Chem. 42(15), 3001-3003 (1999) and published PCT Application WO 01/18171 published on March 15, 2001 to Sloan-Kettering Institute for Cancer Research and The Trustees of Columbia University. The entire teachings of all of the above are hereby incorporated by reference.

Some notable hydroxamic acid based HDAC inhibitors are suberoylanilide hydroxamic acid (SAHA), m-carboxycinnamic acid bishydroxamate (CBHA) and pyroxamide. SAHA has been shown to bind directly in the catalytic pocket of the histone deacetylase enzyme. SAHA induces cell cycle arrest, differentiation and/or apoptosis of transformed cells in culture and inhibits tumor growth in rodents. SAHA is effective at inducing these effects in both solid tumors and hematological cancers. It has been shown that SAHA is effective at inhibiting tumor growth in animals with no toxicity to the animal. The SAHA-induced inhibition of tumor growth is associated with an accumulation of acetylated histones in the tumor. SAHA is effective at inhibiting the development and continued growth of carcinogen-induced (N-methylnitrosourea) mammary tumors in rats. SAHA was administered to the rats in their diet over the 130 days of the study. Thus, SAHA is a nontoxic, orally active antitumor agent whose mechanism of action involves the inhibition of histone deacetylase activity.

### SUMMARY OF THE INVENTION

It has been surprisingly discovered that certain HDAC inhibitors, for example, SAHA, CBHA and pyroxamide can cross the blood brain barrier at sufficient amounts to significantly inhibit HDAC activity causing the accumulation of acetylated histones in the brain. This discovery therefore provides for the use of HDAC inhibitors for inhibiting HDAC in the brain, for the treatment of disorders of the central nervous system including cancer of the brain and neurodegenerative diseases.

The present application is directed to a method of inhibiting HDAC in the brain of a mammal. The method comprises administering to an individual a HDAC inhibiting amount of a histone deacetylase inhibitor compound. The present application is further directed to a method of treating diseases of the central nervous system (CNS) comprising administering to a individual in need of treatment a therapeutically effective amount of an inhibitor of HDAC. In particular embodiments, the CNS disease is a neurodegenerative disease. In further embodiments, the neurogenerative disease is an inherited neurodegenerative disease, such as those inherited neurodegenerative diseases which are polyglutamine expansion diseases. In other embodiments, the disorder is cancer of the brain.

The individual is a mammal such as a primate or human.

### BRIEF DESCRIPTION OF THE DRAWINGS

FIG. 1 is a scan of a Western blot and Coomassie stained gel indicating levels of acetylated histone (αAcH3) at the indicated timepoints following treatment with vehicle (DMSO) or three doses of SAHA (100 mg/kg/hr).
FIG. 2 is a scan of a Western blot and Coomassie stained gel indicating levels of acetylated histone (αAcH4) at the indicated timepoints following treatment with vehicle (DMSO) or three doses of Pyroxamide (100 mg/kg/hr).

The foregoing and other objects, features and advantages of the invention will be apparent from the following more particular description of preferred embodiments of the invention, as illustrated in the accompanying drawings.

### DETAILED DESCRIPTION OF THE INVENTION

The present application is directed to a method of inhibiting HDAC in the brain of a mammal. The method comprises administering to an individual a HDAC inhibiting amount of a histone deacetylase inhibitor compound. The present application is further directed to a method of treating diseases of the central nervous system (CNS) comprising administering to a individual in need of treatment a therapeutically effective amount of an inhibitor of HDAC In particular embodiments, the CNS disease is a neurodegenerative disease. In further embodiments, the neurogenerative disease is an inherited neurodegenerative disease, such as those inherited neurodegenerative diseases which are polyglutamine expansion diseases. In a preferred embodiment, the neurodegenerative disease is Huntington's disease. In yet other embodiments, the disorder is cancer of the brain.

The individual is a mammal such as a primate or human.

Therapeutically effective amount as that term is used herein refers to an amount which elicits the desired therapeutic effect. The therapeutic effect is dependent upon the disease being treated and the results desired. As such, the therapeutic effect can be a decrease in the severity of symptoms associated with the disease and/or inhibition (partial or complete) of progression of the disease. Further, the therapeutic effect can be inhibition of HDAC in the brain. The amount needed to elicit the therapeutic response can be determined based on the age, health, size and sex of the patient. Optimal amounts can also be determined based on monitoring of the patient's response to treatment.

Generally, diseases of the central nervous system, are referred to as neurodegenerative, indicating that they are characterized by gradually evolving, relentlessly progressive neuronal death with many occurring for reasons that are still largely unknown. The identification of these diseases depends upon exclusion of such possible causative factors as infections, metabolic derangements, and intoxications. A considerable proportion of the disorders classed as neurogenerative are genetic, with either dominant or recessive inheritance. Others, however, occur only sporadically as isolated instances in a given family. Classification of the degenerative diseases cannot be based upon any exact knowledge of cause or pathogenesis; their subdivision into individual syndromes rests on descriptive criteria based largely upon neuropathologic and clinical aspects.

However, research in the past decade has uncovered a new classification of inherited neurodegenerative diseases, the polyglutamine (polyQ) expansion diseases. In each, the underlying mutation is an expansion of a CAG trinucleotide repeat that encodes polyQ in the respective disease protein. All are progressive, ultimately fatal disorders that typically begin in adulthood and progress over 10 to 30 years. The clinical features and pattern of neuronal degeneration differ among the diseases, yet increasing evidence suggests that polyQ diseases share important pathogenic features. In particular, abnormal protein conformations promoted by polyQ expansion seem to be central to pathogenesis. This class of PolyQ expansion neurodegenerative disease are Huntington's Disease (HD), Dentatorubralpallidoluysian atrophy (DRPLA), spinal and bulbar muscular atrophy (SBMA), and five spinocerebellar ataxias (SCA1, SCA2, SCA3/MJD(Machado-Joseph Disease), SCA6 and SCA7). These diseases are listed in the general listing of neurodegenrative disease below. Many of these diseases not yet connected with PolyQ expansion are thought to result from abnormal protein folding and aggregation (*e.g*., Alzheimer's disease).

Generally, neurodegenerative diseases can be grouped as follows:
I. Disorders characterized by progressive dementia in the absence of other prominent neurologic signs.
   A. Alzheimer's disease
   B. Senile dementia of the Alzheimer type
   C. Pick's disease (lobar atrophy)
II. Syndromes combining progressive dementia with other prominent neurologic abnormalities
   A. Mainly in adults
      1. Huntington's disease
      2. Multiple system atrophy combining dementia with ataxia and/or manifestations of Parkinson's disease
      3. Progressive supranuclear aplsy (Steel-Richardson-Olszewski)
      4. Diffuse Lewy body disease
      5. Corticodentatonigral degeneration
   B. Mainly in children or young adults
      1. Hallervorden-Spatz disease
      2. Progressive familial myoclonic epilepsy
III. Syndromes of gradually developing abnormalities of posture and movement
   A. Paralysis agitans (Parkinson's disease)
   B. Striatonigral degeneration
   C. Progressive supranuclear palsy
   D. Torsion dystonia (torsion spasm; dystonia musculorum deformans)
   E. Spasmodic torticollis and other dyskinesis
   F. Familial tremor
   G. Gilles de la Tourette syndrome
IV. Syndromes of progressive ataxia
   A. Cerebellar degenerations
      1. Cerebellar cortical degeneration
      2. Olivopontocerebellar atrophy (OPCA)
   B. Spinocerebellar degeneration (Friedreich's atazia and related disorders)
V. Syndrome of central autonomic nervous system failure (Shy-Drager syndrome)
VI. Syndromes of muscular weakness and wasting without sensory changes (motor neuron disease)
   A. Amyotrophic lateral sclerosis
   B. Spinal muscular atrophy
      1. Infantile spinal muscular atrophy (Werdnig-Hoffman)
      2. Juvenile spinal muscular atrophy (Wohlfart-Kugelberg-Welander)
      3. Other forms of familial spinal muscular atrophy
   C. Primary lateral sclerosis
   D. Hereditary spastic paraplegia
VII. Syndromes combining muscular weakness and wasting with sensory changes (progressive neural muscular atrophy; chronic familial polyneuropathies)
   A. Peroneal muscular atrophy (Charcot-Marie-Tooth)
   B. Hypertrophic interstitial polyneuropathy (Dejerine-Sottas)
   C. Miscellaneous forms of chronic progressive neuropathy
VIII. Syndromes ofprogressive visual loss
   A. Pigmentary degeneration of the retina (retinitis pigmentosa)
   B. Hereditary optic atrophy (Leber's disease)

As used herein, "brain cancer" refers to cancer resulting from both primary and metastatic brain tumors. Gliomas are primary brain tumors which arise from the glial cells in the brain and spinal cord, and are the most common primary brain tumors. Gliomas are classified into several groups based on the type of glial cell involved. For example, astrocytomas, which are the most common type of gliomas, are developed from astrocytes. Types of astrocytomas include well-differentiated, anaplastic, and glioblastoma multiforme. Other types of glioma include ependymomas, oligodendrogliomas, ganglioneuromas, mixed gliomas, brain stem gliomas, optic nerve gliomas, meningiomas, pineal tumors, pituitary adenomas, and primitive neuroectodermal tumors, such as medulloblastomas, neuroblastomas, pineoblastomas, medulloepitheliomas, ependymoblastomas and polar spongioblastomas. Non-glioma type tumors include chordomas and craniopharyngiomas.

Histone deacetylases (HDACs) as that term is used herein are enzymes which catalyze the removal of acetyl groups from lysine residues in the amino terminal tails of the nucleosomal core histones. As such, HDACs together with histone acetyl transferases (HATs) regulate the acetylation status of histones. Histone acetylation affects gene expression and inhibitors of HDACs, such as the hydroxamic acid-based hybrid polar compound suberoylanilide hydroxamic acid (SAHA) induce growth arrest, differentiation and/or apoptosis of transformed cells *in vitro* and inhibit tumor growth *in vivo.* HDACs can be divided into three classes based on structural homology. Class I HDACs (HDACs 1, 2, 3 and 8) bear similarity to the yeast RPD3 protein, are located in the nucleus and are found in complexes associated with transcriptional corepressors. Class II HDACs (HDACs 4, 5, 6, 7 and 9) are similar to the yeast HDA1 protein, and have both nuclear and cytoplasmic subcellular localization. Both Class I and II HDACs are inhibited by hydroxamic acid-based HDAC inhibitors, such as SAHA. Class III HDACs form a structurally distant class of NAD dependent enzymes that are related to the yeast SIR2 proteins and are not inhibited by hydroxamic acid-based HDAC inhibitors.

Histone deacetylase inhibitors or HDAC inhibitors, as that term is used herein are compounds which are capable of inhibiting the deacetylation of histones *in vivo*, *in vitro* or both. As such, HDAC inhibitors inhibit the activity of at least one histone deacetylase. As a result of inhibiting the deacetylation of at least one histone, an increase in acetylated histone occurs and accumulation of acetylated histone is a suitable biological marker for assessing the activity of HDAC inhibitors. Therefore, procedures which can assay for the accumulation of acetylated histones can be used to determine the HDAC inhibitory activity of compounds of interest. It is understood that compounds which can inhibit histone deacetylase activity can also bind to other substrates and as such can inhibit other biologically active molecules such as enzymes. It is also understood, that HDAC inhibitors suitable for use in the invention described herein are capable of crossing the Blood Brain Barrier.

For example, in patients receiving HDAC inhibitors, the accumulation of acetylated histones in peripheral mononuclear cells as well as in tissue treated with HDAC inhibitors can be determined against a suitable control.

HDAC inhibitory activity of a particular compound can be determined *in vitro* using, for example, an enzymatic assays which shows inhibition of at least one histone deacetylase. Further, determination of the accumulation of acetylated histones in cells treated with a particular composition can be determinative of the HDAC inhibitory activity of a compound.

Assays for the accumulation of acetylated histones are well known in the literature. See, for example, Marks, P.A. et al., J. Natl. Cancer Inst., 92:1210-1215, 2000, Butler, L.M. et al., Cancer Res. 60:5165-5170 (2000), Richon, V. M. et al., Proc. Natl. Acad. Sci., USA, 95:3003-3007, 1998, and Yoshida, M. et al., J. Biol. Chem., 265:17174-17179,1990.

For example, an enzymatic assay to determine the activity of a histone deacetylase inhibitor compound can be conducted as follows. Briefly, the effect of an HDAC inhibitor compound on affinity purified human epitope-tagged (Flag) HDAC1 can be assayed by incubating the enzyme preparation in the absence of substrate on ice for about 20 minutes with the indicated amount of inhibitor compound. Substrate ([³H]acetyl-labelled murine erythroleukemia cell-derived histone) can be added and the sample can be incubated for 20 minutes at 37°C in a total volume of 30 µL. The reaction can then be stopped and released acetate can be extracted and the amount of radioactivity release determined by scintillation counting. An alternative assay useful for determining the activity of a histone deacetylase inhibitor compound is the "HDAC Fluorescent Activity Assay;Drug Discovery Kit-AK-500" available from BIOMOL® Research Laboratories, Inc., Plymouth Meeting, PA.

In vivo studies can be conducted as follows. Animals, for example mice, can be injected intraperitoneally with an HDAC inhibitor compound. Selected tissues, for example brain, spleen, liver etc..., can be isolated at predetermined times, post administration. Histones can be isolated from tissues essentially as described by Yoshida et al., J. Biol. Chem. 265:17174-17179, 1990. Equal amounts of histones (about 1 µg) can be electrophoresed on 15% SDS-polyacrylamide gels and can be transferred to Hybond-P filters (available from Amersham). Filters can be blocked with 3% milk and can be probed with a rabbit purified polyclonal anti-acetylated histone H4 antibody (αAc-H4) and anti-acetylated histone H3 antibody (αAc-H3) (Upstate Biotechnology, Inc.). Levels of acetylated histone can be visualized using a horseradish peroxidase-conjugated goat anti-rabbit antibody (1:5000) and the SuperSignal chemiluminescent substrate (Pierce). As a loading control for the histone protein, parallel gels can be run and stained with Coomassie Blue (CB).

In addition, hydroxamic acid-based HDAC inhibitors have been shown to up regulate the expression of the p21^{WAF1} gene. The p21^{WAF1} protein is induced within 2 hours of culture with HDAC inhibitors in a variety of transformed cells using standard methods. The induction of the p21^{WAF1} gene is associated with accumulation of acetylated histones in the chromatin region of this gene. Induction of p21^{WAF1} can therefore be recognized as involved in the G1 cell cycle arrest caused by HDAC inhibitors in transformed cells.

Typically, HDAC inhibitors fall into four general classes: 1) short-chain fatty acids (*e*.*g*., 4-phenylbutyrate and valproic acid); hydroxamic acids (*e*.*g*., SAHA, Pyroxamide, trichostatin A (TSA), oxamflatin, Scriptaid and CHAPs, such as, CHAP1 and CHAP 31); 3) cyclic tetrapeptides (Trapoxin A and Apicidin; 4) benzamides (*e.g*., MS-275). Examples of such compounds can be found in U.S. Patent Nos. 5,369,108, issued on November 29, 1994, 5,700,811, issued on December 23, 1997, 5,773,474, issued on June 30,1998 and 5,932,616 issued onAugust 3, 1999 all to Breslow et al., U.S. Patent Nos. 5,055,608, issued on October 8, 1991, 5,175,191, issued on December 29, 1992 and 5,608,108 issued on March 4, 1997 all to Marks et al., as well as, Yoshida, M., et al., Bioassays 17, 423-430 (1995), Saito, A., et al., PNAS USA 96, 4592-4597, (1999), Furamai R. et al., PNAS USA 98 (1), 87-92 (2001), Komatsu, Y., et al., Cancer Res. 61(11), 4459-4466 (2001), Su, G.H., et al., Cancer Res. 60, 3137-3142 (2000), Lee, B.I. et al., Cancer Res. 61(3), 931-934, Suzuki, T., et al., J. Med. Chem. 42(15), 3001-3003 (1999), published PCT Application WO 01/18171 published on March 15, 2001 to Sloan-Kettering Institute for Cancer Research and The Trustees of Columbia University, published PCT Applications WO 01/38322 (published May 31, 2001), WO 01/70675 (published on September 27,2001) and WO 00/71703 (published on November 30, 2000) all to Methylgene, Inc., published PCT Application WO 00/21979 published on October 8, 1999 to Fujisawa Pharmaceutical Co., Ltd. an published PCT Application WO 98/40080 published on March 11, 1998 to Beacon Laboratories, L.L.C. the entire content of all of which are hereby incorporated by reference. It is understood that HDAC inhibitors suitable for use in the invention described herein must be capable of permeabiliziing the blood brain barrier such that therapeutic levels of inhibitor can enter the CNS.

Preferred hydroxamic acid based HDAC inhibitor are suberoylanilide hydroxamic acid (SAHA), m-carboxycinnamic acid bishydroxamate (CBHA) and pyroxamide. SAHA has been shown to bind directly in the catalytic pocket of the histone deacetylase enzyme. SAHA induces cell cycle arrest, differentiation and/or apoptosis of transformed cells in culture and inhibits tumor growth in rodents. SAHA is effective at inducing these effects in both solid tumors and hematological cancers. It has been shown that SAHA is effective at inhibiting tumor growth in animals with no toxicity to the animal. The SAHA-induced inhibition of tumor growth is associated with an accumulation of acetylated histones in the tumor. SAHA is effective at inhibiting the development and continued growth of carcinogen-induced (N-methylnitrosourea) mammary tumors in rats. SAHA was administered to the rats in their diet over the 130 days of the study. Thus, SAHA is a nontoxic, orally active antitumor agent whose mechanism of action involves the inhibition of histone deacetylase activity.

SAHA can be represented by the following structural formula:

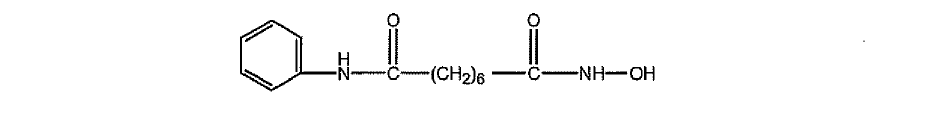

Pyroxamide can be represented by the following structural formula:

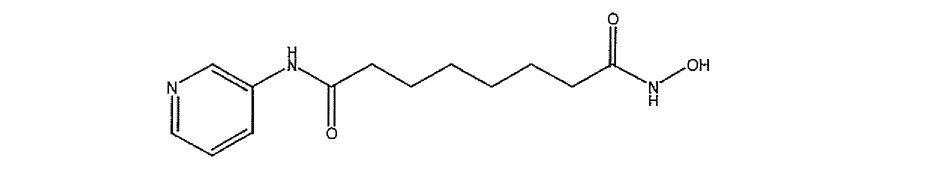

CBHA can be represented by the structural formula:

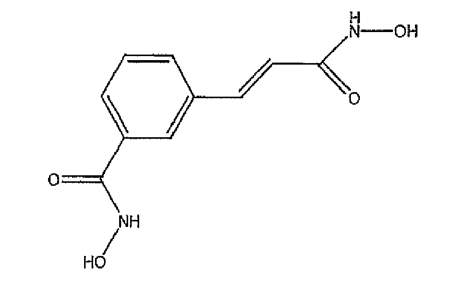

In one embodiment, the HDAC inhibitor can be represented by Formula I: 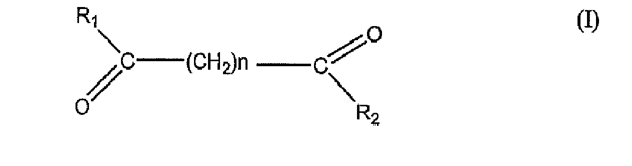 wherein R₁ and R₂ can be the same or different; when R₁ and R₂ are the same, each is a substituted or unsubstituted arylamino, cycloalkylamino, pyridineamino, piperidino, 9-purine-6-amine or thiazoleamino group; when R₁ and R₂ are different R₁=R₃-N-R₄, wherein each of R₃ and R₄ are independently the same as or different from each other and are a hydrogen atom, a hydroxyl group, a substituted or unsubstituted, branched or unbranched alkyl, alkenyl, cycloalkyl, aryl alkyloxy, aryloxy, arylalkyloxy or pyridine group, or R₃ and R₄ bonded together to form a piperidine group, R₂ is a hydroxylamino, hydroxyl, amino, alkylamino, dialkylamino or alkyloxy group and n is an integer from about 4 to about 8.

As such, in another embodiment the HDAC inhibitors used in the method of the invention can be represent Formula II: 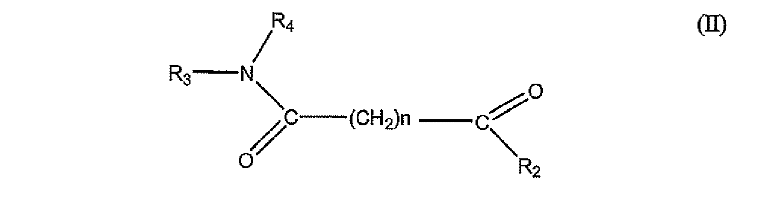 wherein each of R₃ and R₄ are independently the same as or different from each other and are a hydrogen atom, a hydroxyl group, a substituted or unsubstituted, branched or unbranched alkyl, alkenyl, cycloalkyl, arylalkyloxy, aryloxy, arylalkyloxy or pyridine group, or R₃ and R₄ bonded together to form a piperidine group, R₂ is a hydroxylamino, hydroxyl, amino, alkylamino, dialkylamino or alkyloxy group and n is an integer from about 4 to about 8.

In a particular embodiment of Formula II, R₂ is a hydroxylamino, hydroxyl, amino, methylamino, dimethylamino or methyloxy group and n is 6. In yet another embodiment of Formula II, R₄ is a hydrogen atom, R₃ is a substituted or unsubstituted phenyl and n is 6. In further embodiments of Formula II, R₄ is hydrogen and R₃ is an α-, β-, or γ-pyridine.

In other specific embodiments of Formula II, R₄ is a hydrogen atom and R₃ is a cyclohexyl group; R₄ is a hydrogen atom and R₃ is a methoxy group; R₃ and R₄ each bond together to form a piperidine group; R₄ is a hydrogen atom and R₃ is a hydroxyl group; R₃ and R₄ are both a methyl group and R₃ is phenyl and R₄ is methyl.

Further HDAC inhibitors suitable for use in the present invention can be represented by structural Formula III: 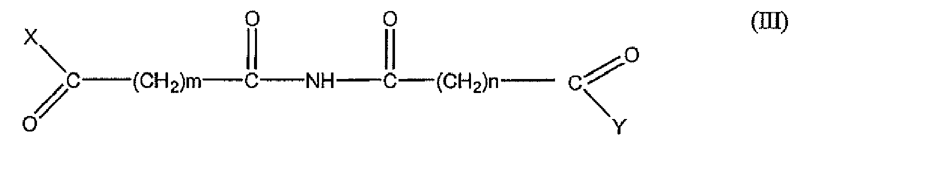 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino, or aryloxyalkylamino group; R is a hydrogen atom, a hydroxyl, group, a substituted or unsubstituted alkyl, arylalkyloxy, or aryloxy group; and each of m and n are independently the same as or different from each other and are each an integer from about 0 to about 8.

In a particular embodiment, the HDAC inhibitor is a compound of Formula III wherein X, Y and R are each hydroxyl and both m and n are 5.

In yet another embodiment, the HDAC inhibitor compounds suitable for use in the method of the invention can be represented by structural Formula IV: 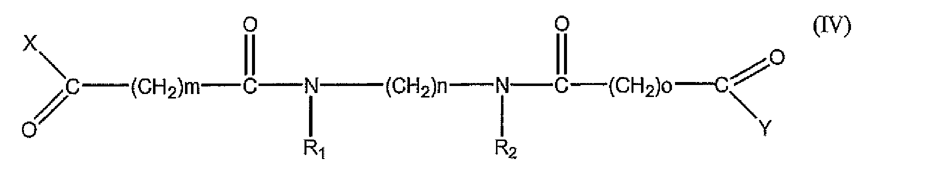 wherein each of X and Y are independently the same as or different from each other and are a hydroxyl, amino or hydroxylamino group, a substituted or unsbustituted aryloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m, n and o are independently the same as or different from each other and are each an integer from about 0 to about 8.

Other HDAC inhibitors suitable for use in the invention include compounds having structural Formula V: 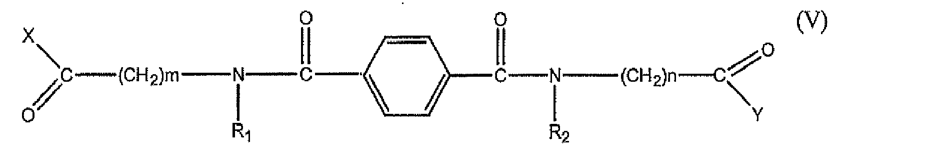 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m and n are independently the same as or different from each other and are each an integer from about 0 to about 8.

In a further embodiment, HDAC inhibitors suitable for use in the method of the present invention can have structural Formula VI: 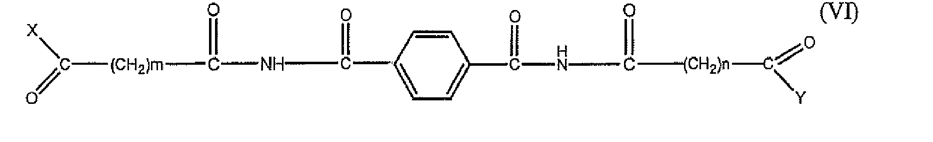 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; and each of m and n are independently the same as or different from each other and are each an integer from about 0 to about 8.

In yet another embodiment, the HDAC inhibitors useful in the method of the invention can have structural Formula VII: 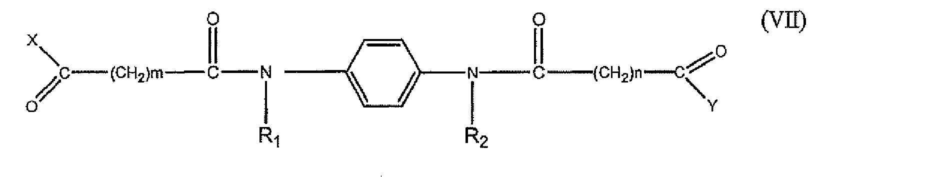 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; R₁ and R₂ are independently the same as or different from each other and are a hydrogen atom, a hydroxyl group, a substituted or unsubstituted alkyl, arylalkyloxy or aryloxy group; and each of m and n are independently the same as or different from each other and are each an integer from about 0 to about 8.

In yet a further embodiment, HDAC inhibitors suitable for use in the invention can have structural Formula VIII: 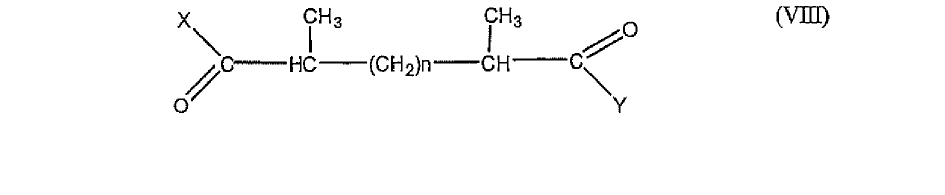 wherein each of X an Y are independently the same as or different from each other and are a hydroxyl, amino or hydroxylamino group, a substituted or unsubstituted alkyloxy, alkylamino, dialkylamino, arylamino, alkylarylamino, or aryloxyalkylamino group; and n is an integer from about 0 to about 8.

Additional compounds suitable for use in the method of the invention include those represented by Formula IX: 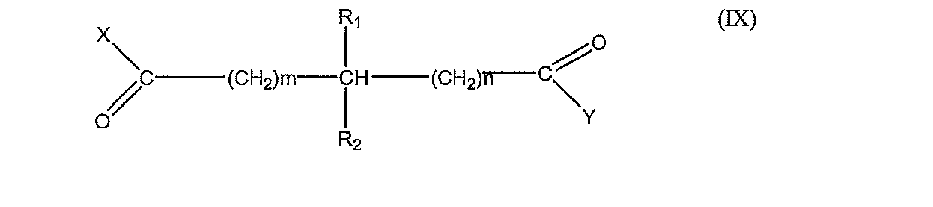 wherein Each of X and Y are independently the same as or different from each other and are a hydroxyl, amino or hydroxylamino group, a substituted or unsbustituted alkyloxy, alkylamino, dialkylamino, arylamino, alkylarylamino, alkylowyamino, aryloxyamino, alkyloxyalkyamino or aryloxyakylamino group; each of R₁ and R₂ are independently the same as or different from each other and are a hydrogen atom, a hydroxyl group, a substitituted or unsubstituted alkyl, aryl, alkyloxy, aryloxy, carbonylhydroxylamino or fluoro group; and each of m and n are independently the same as or different from each other and are each an integer from about 0 to about 8.

In a further embodiment, HDAC inhibitors suitable for use in the invention include compounds having structural Formula X: 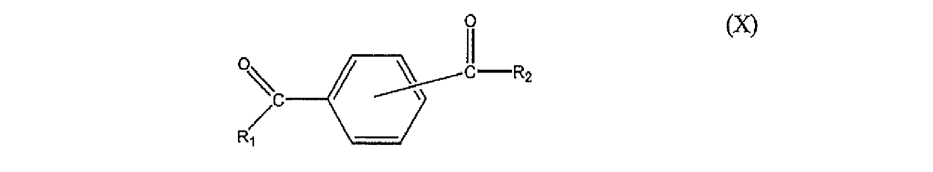 wherein each of R₁ and R₂ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group. In a particular embodiment, the HDAC inhibitor is a compound of structural Formula X wherein R₁ and R₂ are both hydroxylamino.

In a further embodiment, the HDAC inhibitor suitable for use in the invention has structural Formula XI: 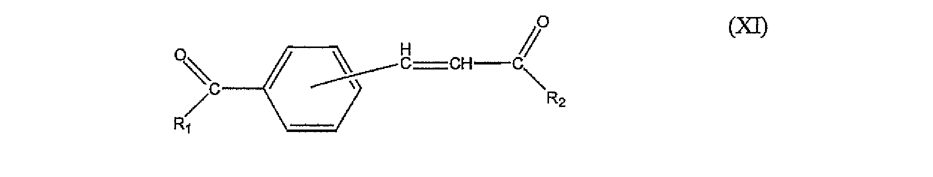 wherein each of R₁ and R₂ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group. In a particular embodiment, the HDAC inhibitor is a compound of structural Formula XI wherein R₁ and R₂ are both hydroxylamino.

In a further embodiment, HDAC inhibitors suitable for use in the present invention include compounds represented by structural Formula XII: 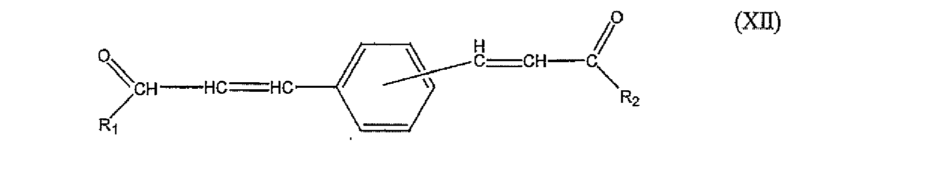 wherein each of R₁ and R₂ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group. In a particular embodiment, the HDAC inhibitor is a compound of structural Formula XII wherein R₁ and R₂ are both hydroxylamino.

Additional compounds suitable for use in the method of the invention include those represented by structural Formula XIII: 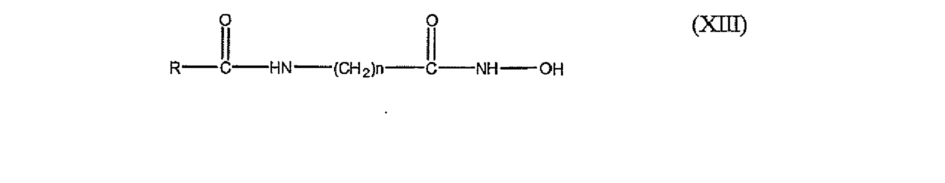 wherein R is a substituted or unsbustituted phenyl, piperidine, thiazole, 2-pyridine, 3-pyridine or 4-pyridine and n is an integer from about 4 toabout 8.

In yet another embodiment, the HDAC inhibitors suitable for use in the method of the invention can be represented by structural Formula (XIV): 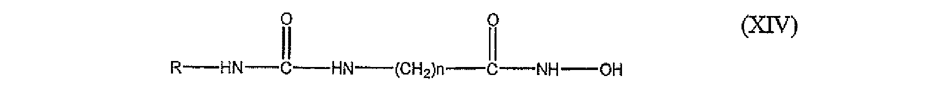 wherein R is a substituted or unsubstituted phenyl, pyridine, piperidine or thiazole group and n is an integer from about 4 to aobut 8 or a pharmaceutically acceptable salt thereof. In a particular embodiment, R is phenyl and n is 5. In another embodiment, n is 5 and R is 3-chlorophenyl.

In structural formulas I-XIV, substituted phenyl, refers to a phenyl group which can be substituted with, for example, but not limited to a methyl, cyano, nitro, trifluoromethyl, amino, aminocarbonyl, methylcyano, halogen, e.g., chloro, fluoro, bromo, iodo, 2,3-difluoro, 2,4-difluoro, 2,5-difluoro, 3,4-difluoro, 3,5-difluoro, 2,6-difluoro, 1,2,3-trifluoro, 2,3,6-trifluoro, 2,3,4,5,6-pentafluoro, azido, hexyl, t-butyl, phenyl, carboxyl, hydroxyl, methyloxy, benzyloxy, phenyloxy, phenylaminooxy, phenylaminocarbonyl, methyloxycarbonyl, methylaminocarbonyl, dimethylamino, dimethylaminocarbonyl or hydroxyaminocarbonyl group.

Other HDAC inhibitors useful in the present invention can be represented by structural Formula XV: 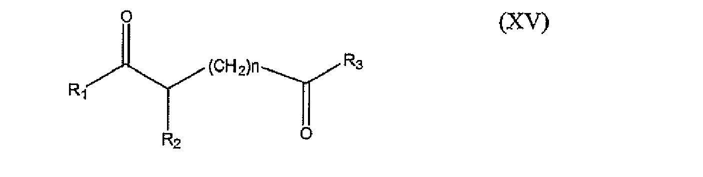 wherein each of R₁ and R₂ is directly attached or through a linker and is substituted or unsubstituted, aryl (e.g. naphthyl, phenyl), cycloalkyl, cycloalkylamino, pyridineamino, piperidino, 9-purine-6-amine, thiazoleamino group, hydroxyl, branched or unbranched alkyl, alkenyl, alkyloxy, aryloxy, arylalkyloxy, or pyridine group; n is an integer from about 3 to about 10 and R₃ is a hydroxamic acid, hydroxylamino, hydroxyl, amino, alkylamino or akyloxy group.

The linker can be an amide moiety, -O-, -S-, -NH- or -CH₂-.

In certain embodiments, R₁ is -NH-R₄ wherein R₄ is substituted or unsubstiuted, aryl (e.g., naphthyl, phenyl), cycloalkyl, cycloalkylamino, pyridineamino, piperidino, 9-purine-6-amine, thiazoleamino group, hydroxyl, branched or unbranched alkyl, alkenyl, alkyloxy, aryloxy, arylalkyloxy or pyridine group.

Further and more specific HDAC inhibitors of Formula XV, include those which can be represented by Formula XVI: 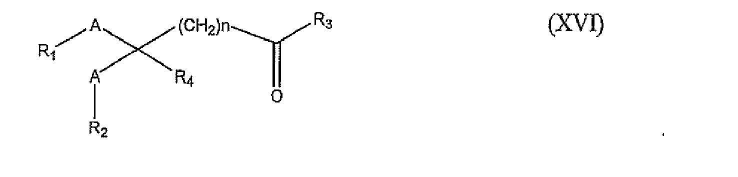 wherein each of R₁ and R₂ is, substituted or unsubstituted, aryl (e.g., phenyl, naphthyl), cycloalkyl, cycloalkylamino, pyridneamino, piperidino, 9-purine-6-amine, thiasoleamino group, hydroxyl, branched or unbranched alkyl, alkenyl, alkyloxy, aryloxy, arylalkyloxy or pyridine group; R₃ is hydroxamic acid, hydroxylamino, hydroxyl, amino, alkylamino or alkyloxy group; R₄ is hydrogen, halogen, phenyl or a cycloalkyl moiety; and A can be the same or different and represents an amide moiety, -O-, -S-, -NR₅- or -CH₂-where R₅ is a substitute or unsubstituted C₁-C₅ alkyl and n is an integer from 3 to 10.

For example, further compounds having a more specific structure within Formula XVI can be represented by structural Formula XVII: 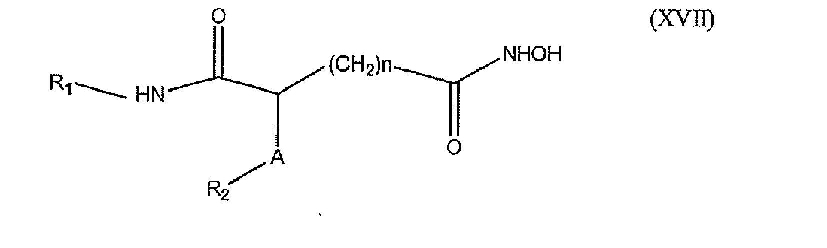 wherein A is an amide moiety, R₁ and R₂ are each selected from substituted or unsubstituted aryl (e.g., pheny, naphthyl), pyridineamino, 9-purine-6-amine, thiazoleamino, aryloxy, arylalkyloxy or pyridine and n is an integer from 3 to 10. For example, the compound can have the formula 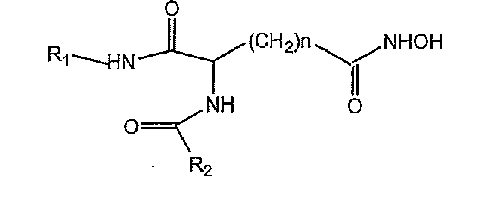 or

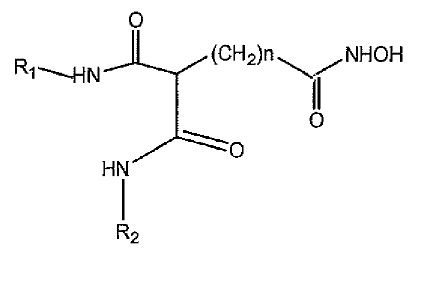

In another embodiment, the HDAC inhibitor can have the Formula XVIII: 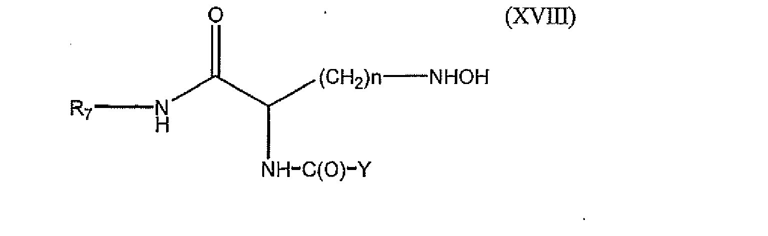 wherein R₇ is selected from substituted or unsubstituted aryl (e.g., phenyl or naphthyl), pyridineamino, 9-purine-6-amine, thiazoleamino, aryloxy, arylakyloxy or pyridine and n is an integer from 3 to 10 and Y is selected from 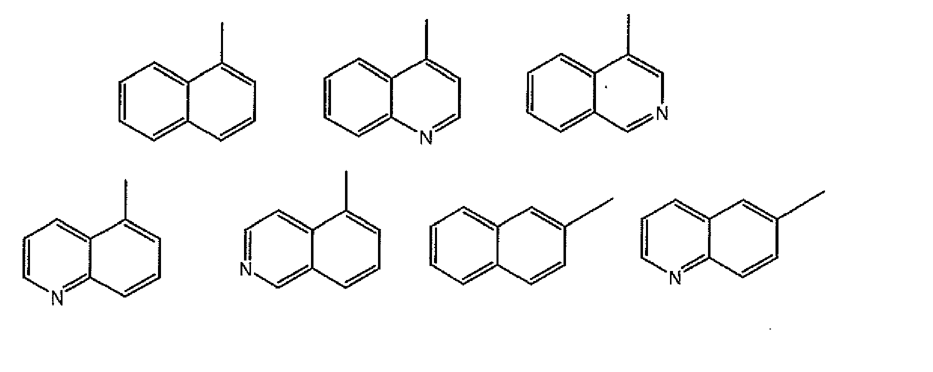 or a pharmaceutically acceptable salt thereof.

In a further embodiment, the HDAC inhibitor compound can have Formula XIX: 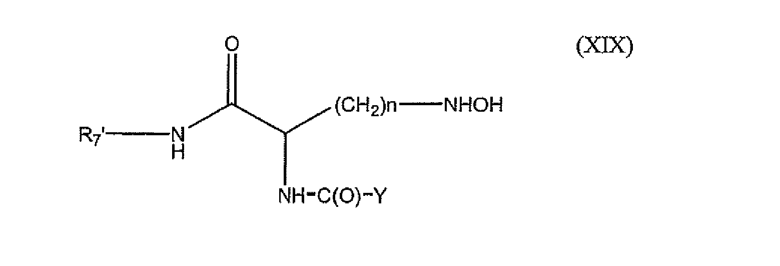 wherein n is an integer from 3 to 10, Y is selected from 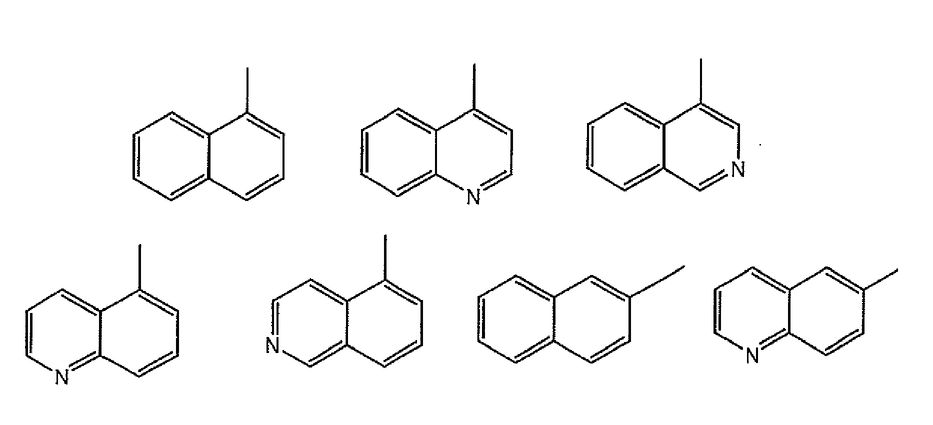 and R₇' is selected from 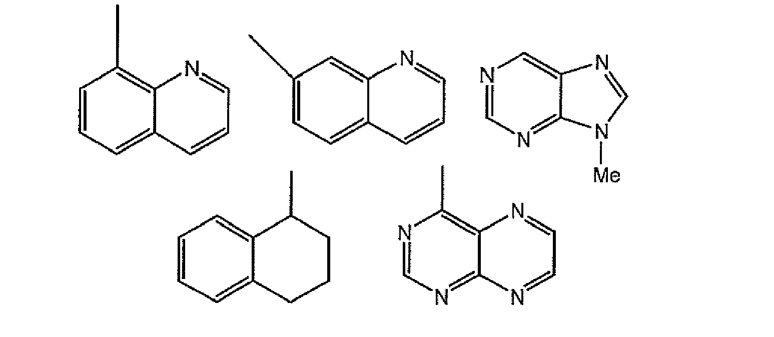 or a pharmaceutically acceptable salt thereof.

Further compounds for use in the invention can be represented by structural Formula XX: 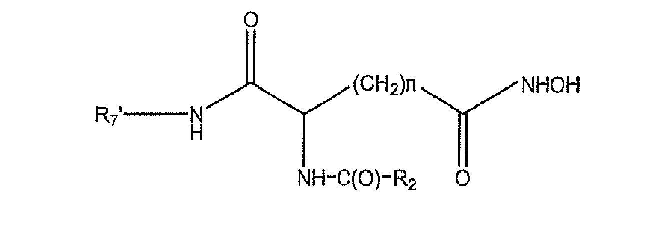 wherein R₂ is selected from substituted or unsubstituted aryl, substituted or unsubstituted naphtha, pyridineamino, 9-purine-6-amine, thiazoleamino, substituted or unsubstituted aryloxy, substituted or unsubstituted arylalkyloxy or pyridine and n is an integer from 3 to 10 and R₇' is selected from 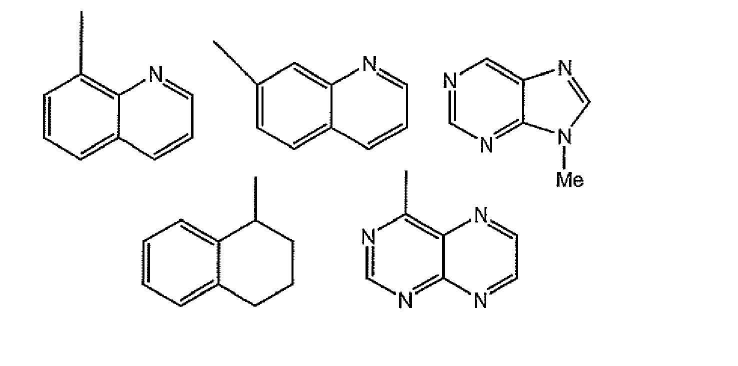 or a pharmaceutically acceptable salt thereof.

Further HDAC inhibitors useful in the invention can be represented by structural Formula XXI: 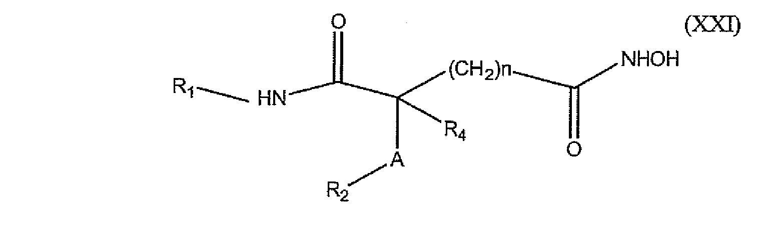 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine, R₄ is hydrogen, a halogen, a phenyl or a cycloalkyl moiety and n is an integer from 3 to 10 or a pharmaceutically acceptable salt thereof.

For example, a compound of Formula XXI can be represented by the structure: 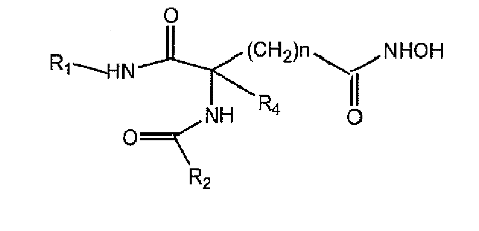 or can be represented by the structure: 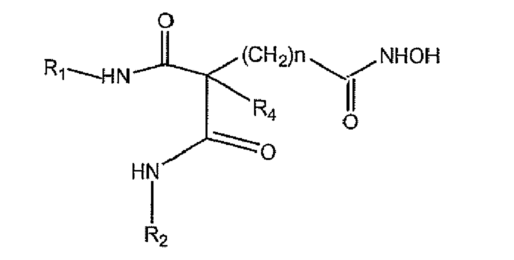 wherein R₁, R₂, R₄ and n have the meanings of Formula XXI.

Further, HDAC inhibitors having the structural Formula XXII: 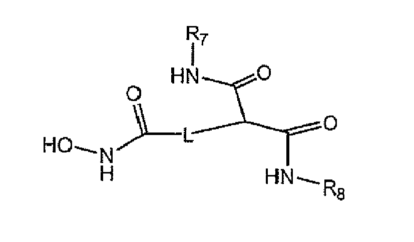 wherein L is a linker selected from the group consisting of -(CH₂)ₙ-, -(CH=CH)ₘ-, - phenyl-, -cycloalkyl-, or any combination thereof; and wherein each of R₇ and R₈ are independently substituted or unsubstituted, aryl, naphtha, pyridineamino, 9-purine-6-amine, thiazoleamino group, aryloxy, arylalkyloxy, or pyridine group, n is an integer from 3 to 10 and m is an integer from 0-10. For example, a compound of Formula XXII can be:

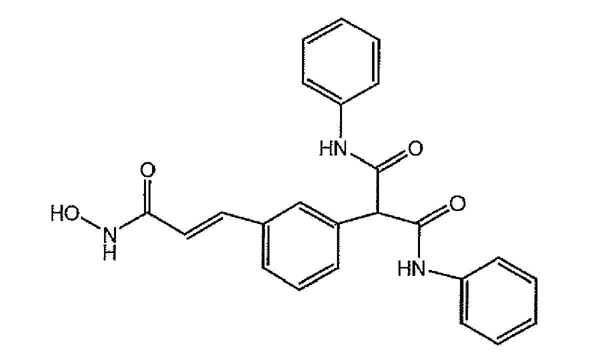

Other HDAC inhibitors suitable for use in the invention include those shown in the following more specific formulas: 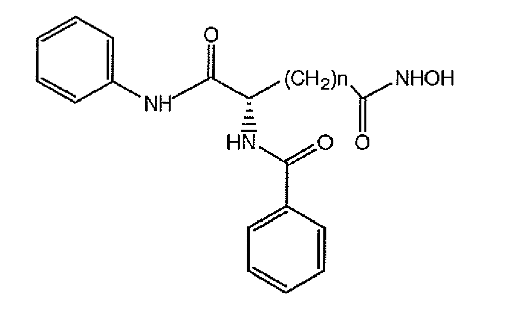 wherein n is an integer from 3 to 10; 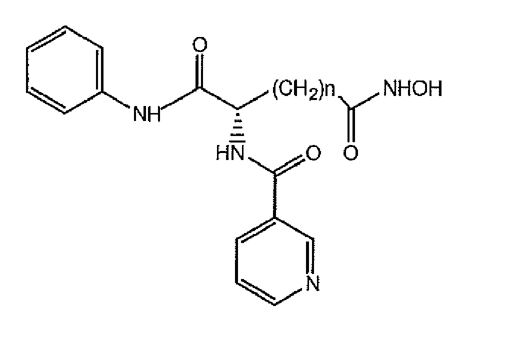 wherein n is an integer from 3 to 10; 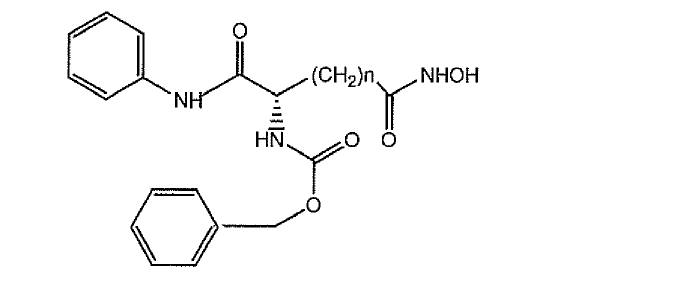 wherein n is an integer from 3 to 10; 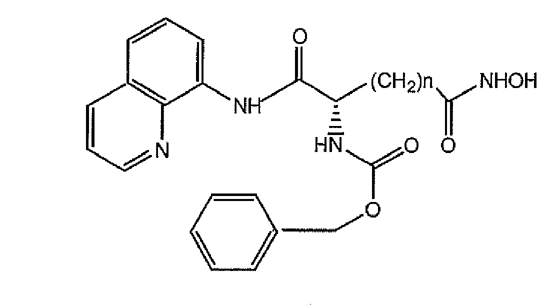 wherein n is an integer from 3 to 10; 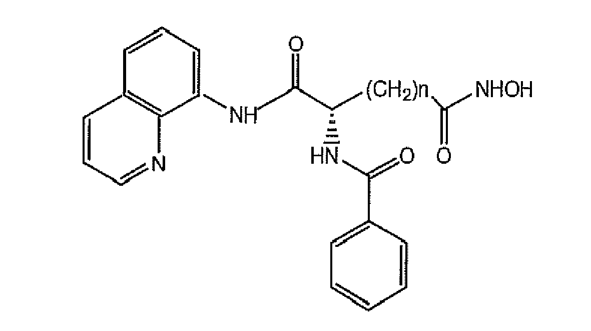 wherein n is an integer from 3 to 10; or a pharmaceutically acceptable salt thereof

Further specific HDAC inhibitors suitable for use in the invention include 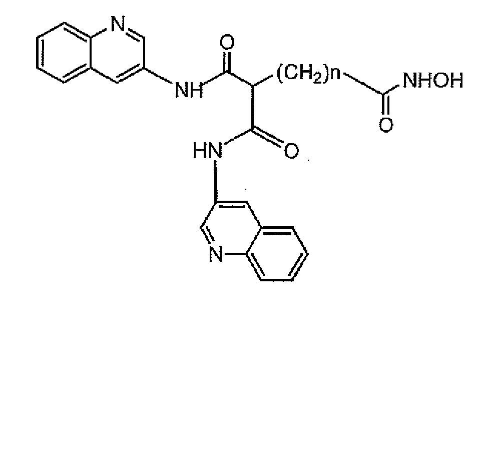 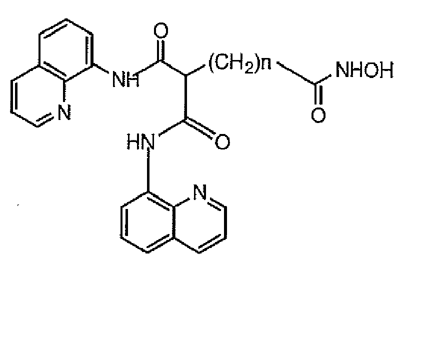 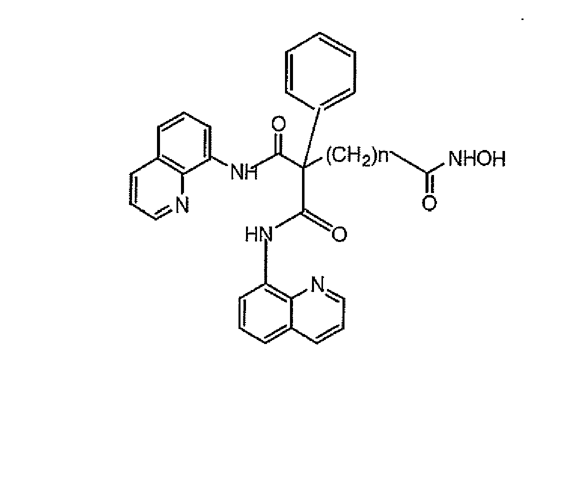 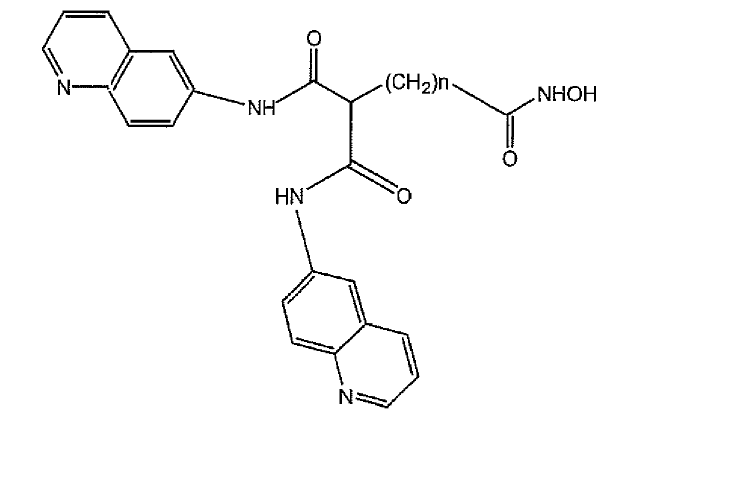 wherein n in each is an integer from 3 to 10 and the compound 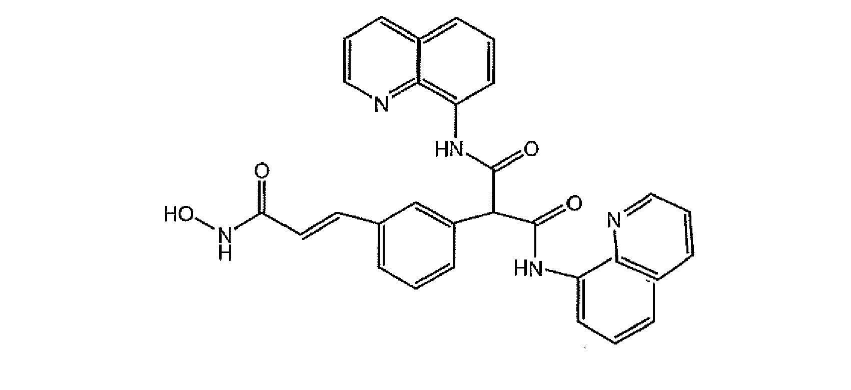 and pharmaceutically acceptable salts thereof.

HDAC inhibitors of the following specific structures are suitable for use in the invention. 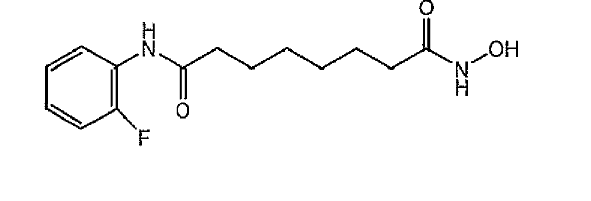 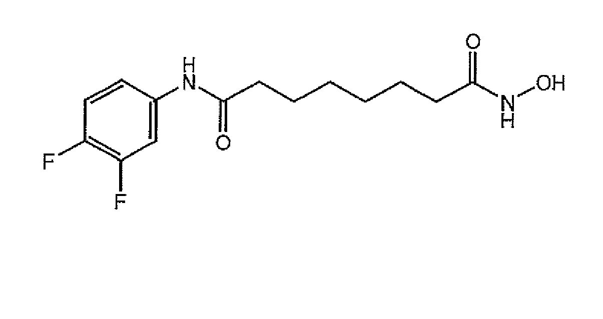 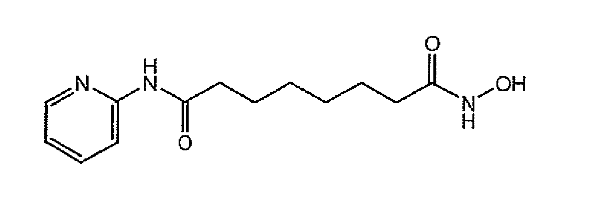 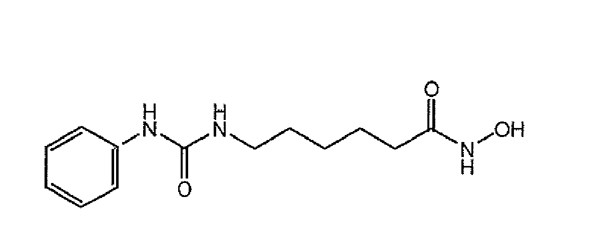 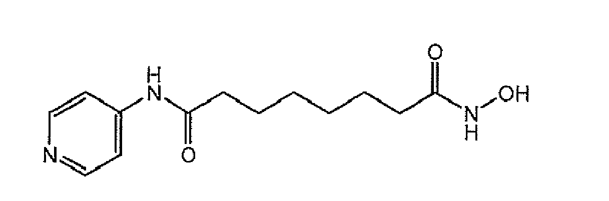 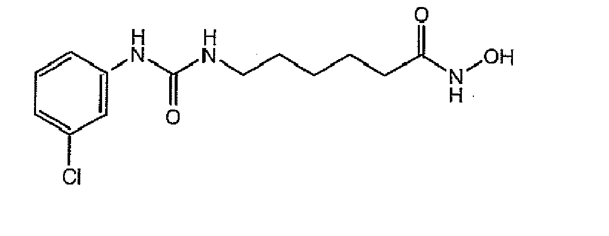 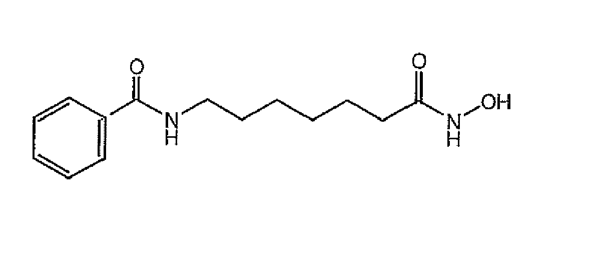 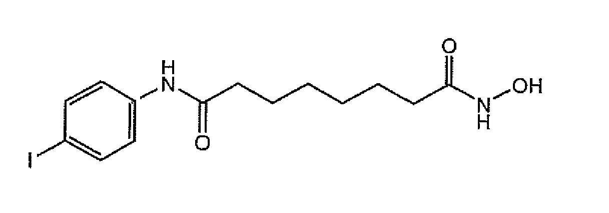 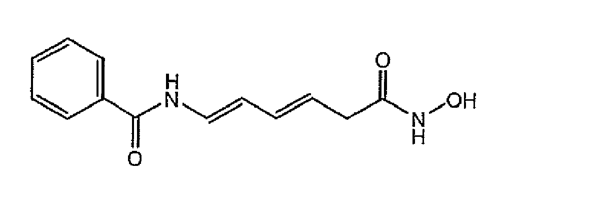 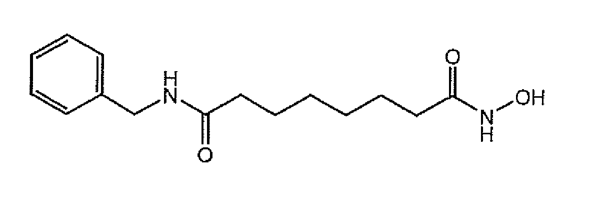 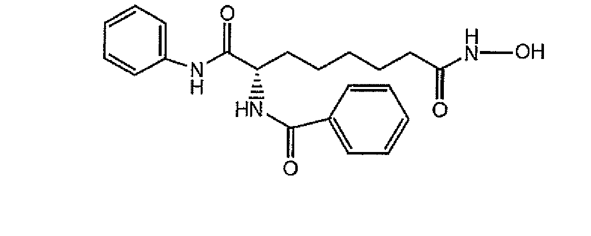 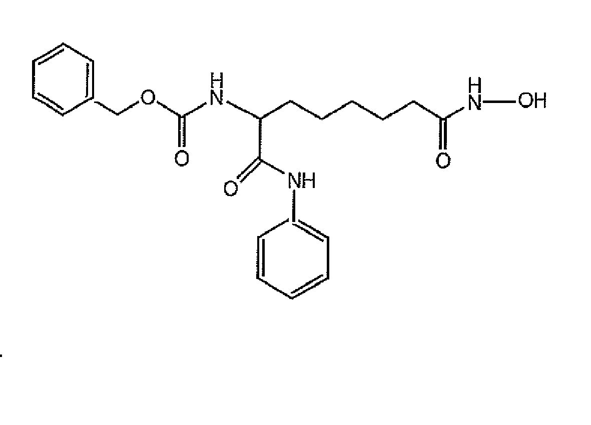 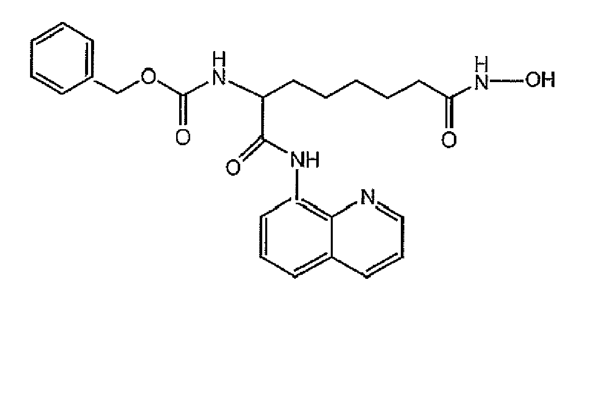 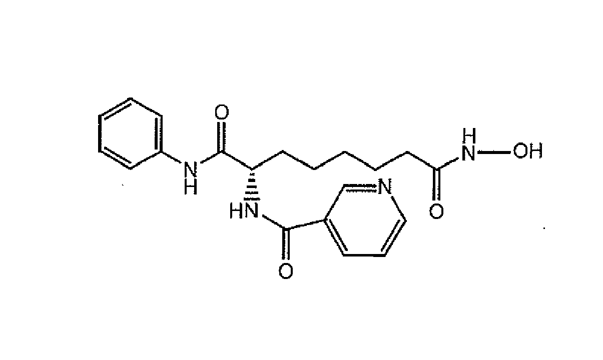 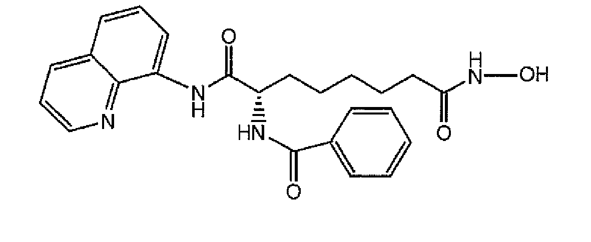 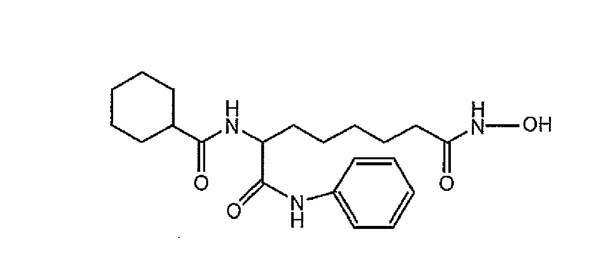 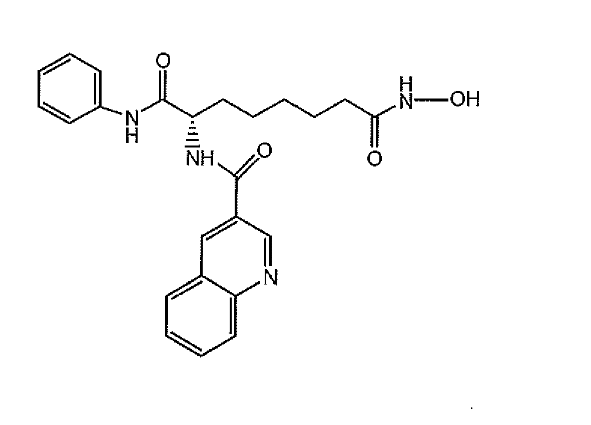 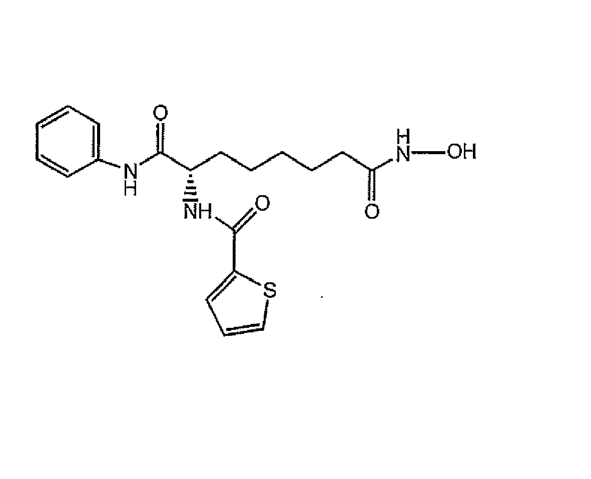 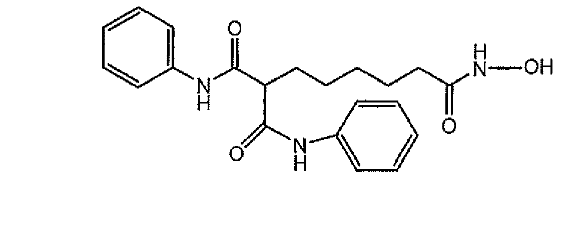 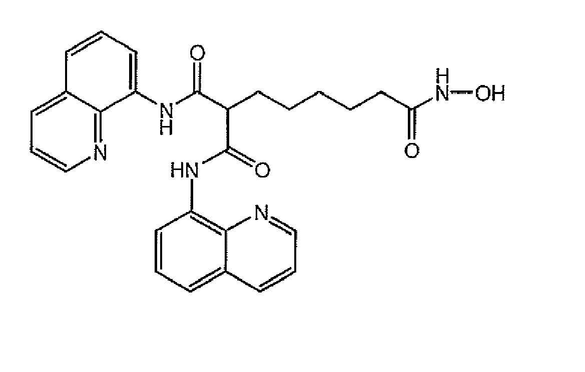 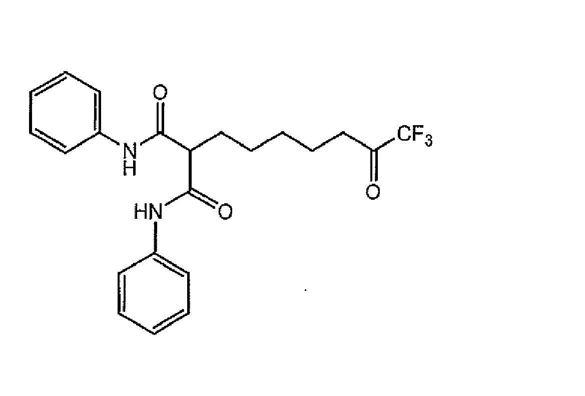 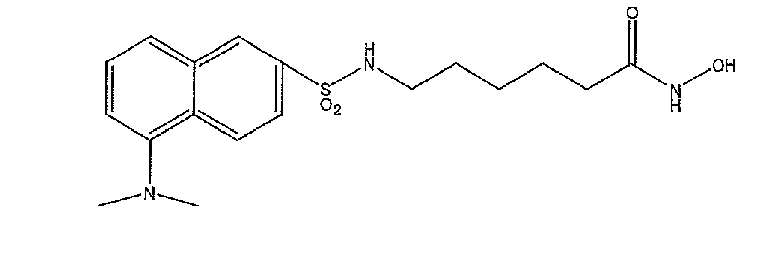 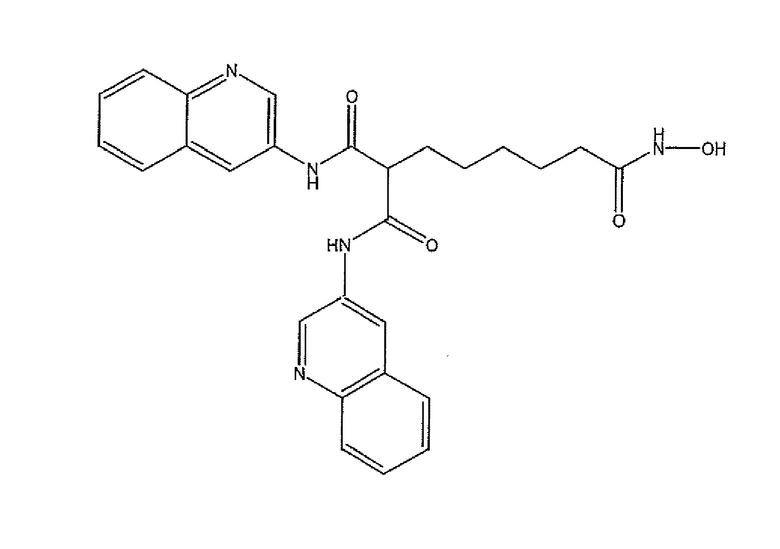 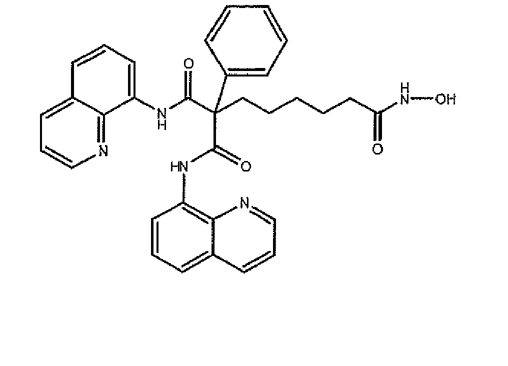 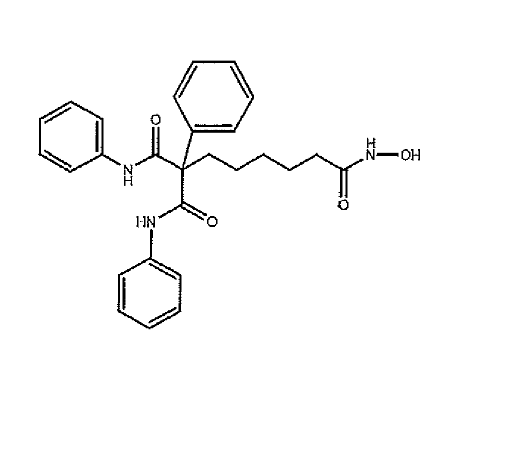 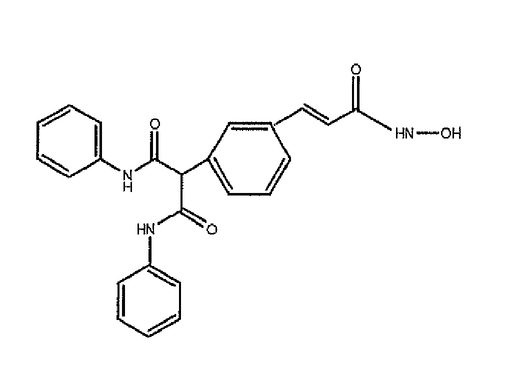 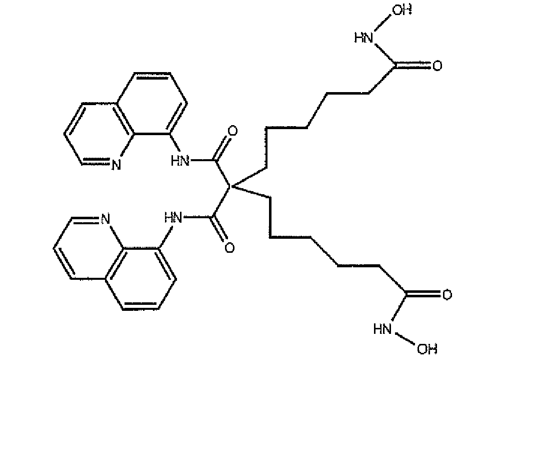 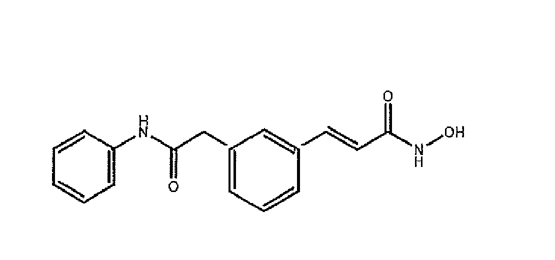 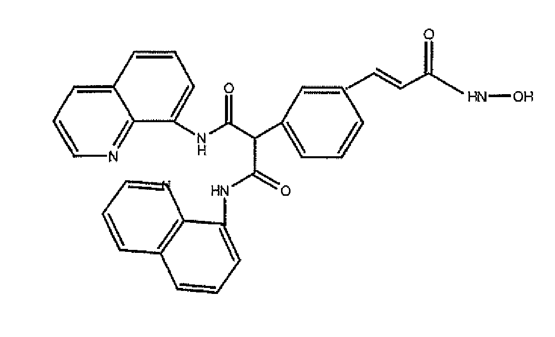 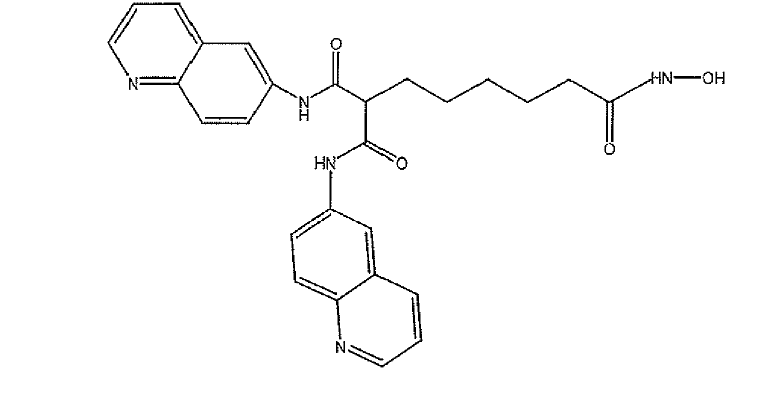 and pharmaceutically acceptable salts thereof.

The HDAC inhibitors of the invention can be administered in such oral forms as tablets, capsules (each of which includes sustained release or timed release formulations), pills, powders, granules, elixers, tinctures, suspensions, syrups, and emulsions. Likewise, the HDAC inhibitors can be administered in intravenous (bolus or infusion), intraperitoneal, subcutaneous, or intramuscular form, all using forms well known to those of ordinary skill in the pharmaceutical arts

The HDAC inhibitors can be administered in the form of a depot injection or implant preparation which can be formulated in such a manner as to permit a sustained release of the active ingredient. The active ingredient can be compressed into pellets or small cylinders and implanted subcutaneously or intramuscularly as depot injections or implants. Implants can employ inert materials such as biodegradable polymers or synthetic silicones, for example, Silastic, silicone rubber or other polymers.

The HDAC inhibitors can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The HDAC inhibitors can also be delivered by the use of monoclonal antibodies as individual carriers to which the compound molecules are coupled.

The HDAC inhibitors can also be prepared with soluble polymers as targetable drug carriers. Such polymers can include polyvinlypyrrolidone, pyran copolymer, polyhydroxy-propyl-methacrylamide-phenol, polyhydroxyethyl-aspartamide-phenol, or polyethyleneoxide-polylysine substituted with palmitoyl residues. Furthermore, the HDAC inhibitors can be prepared with biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

The dosage regimen utilizing the HDAC inhibitors can be selected in accordance with a variety of factors including type, species, age, weight, sex and the disorder being treated; the severity of the condition to be treated; the route of administration; the renal and hepatic function of the patient; and the particular compound or salt thereof employed. An ordinarily skilled physician or veterinarian can readily determine and prescribe the effective amount of the drug required to treat, for example, to prevent, inhibit (fully or partially) or arrest the progress of the disorder.

Oral dosages of the HDAC inhibitors, when used to treat the neurodegenerative diseases described herein or brain cancer, can range between about 2 mg to about 2000 mg per day, such as from about 20 mg to about 2000 mg per day, such as from about 200 mg to about 2000 mg per day. For example, oral dosages can be about 2, about 20, about 200, about 400, about 800, about 1200, about 1600 or about 2000 mg per day. It is understood that the total amount per day can be administered in a single dose or can be administered in multiple dosings such as twice, three or four times per day.

For example, a patient can receive between about 2 mg/day to about 2000 mg/day, for example, from about 20-2000 mg/day, such as from about 200 to about 2000 mg/day, for example from about 400 mg/day to about 1200 mg/day. A suitably prepared medicament for once a day administration can thus contain between about 2 mg and about 2000 mg, such as from about 20 mg to about 2000 mg, such as from about 200 mg to about 1200 mg, such as from about 400 mg/day to about 1200 mg/day. The HDAC inhibitors can be administered in a single dose or in divided doses of two, three, or four times daily. For administration twice a day, a suitably prepared medicament would therefore contain half of the needed daily dose.

Intravenously or subcutaneously, the patient would receive the HDAC inhibitor in quantities sufficient to deliver between about 3-1500 mg/m² per day, for example, about 3, 30, 60, 90, 180, 300, 600, 900, 1200 or 1500 mg/m² per day. Such quantities can be administered in a number of suitable ways, e.g. large volumes of low concentrations of HDAC inhibitor during one extended period of time or several times a day. The quantities can be administered for one or more consecutive days, intermittent days or a combination thereof per week (7 day period). Alternatively, low volumes of high concentrations of HDAC inhibitor during a short period of time, e.g. once a day for one or more days either consecutively, intermittently or a combination thereof per week (7 day period). For example, a dose of 300 mg/m² per day can be administered for 5 consecutive days for a total of 1500 mg/m² per treatment. In another dosing regimen, the number of consecutive days can also be 5, with treatment lasting for 2 or 3 consecutive weeks for a total of 3000 mg/m² and 4500 mg/m² total treatment.

Typically, an intravenous formulation may be prepared which contains a concentration of HDAC inhibitor of between about 1.0 mg/mL to about 10 mg/mL, e.g. 2.0 mg/mL, 3.0 mg/mL, 4.0 mg/mL, 5.0 mg/mL, 6.0 mg/mL, 7.0 mg/mL, 8.0 mg/mL, 9.0 mg/mL and 10 mg/mL and administered in amounts to achieve the doses described above. In one example, a sufficient volume of intravenous formulation can be administered to a patient in a day such that the total dose for the day is between about 300 and about 1500 mg/m².

Glucuronic acid, L-lactic acid, acetic acid, citric acid or any pharmaceutically acceptable acid/conjugate base with reasonable buffering capacity in the pH range acceptable for intravenous administration of the HDAC inhibitor can be used as buffers. Sodium chloride solution wherein the pH has been adjusted to the desired range with either acid or base, for example, hydrochloric acid or sodium hydroxide, can also be employed. Typically, a pH range for the intravenous formulation can be in the range of from about 5 to about 12. A preferred pH range for intravenous formulation wherein the HDAC inhibitor has a hydroxamic acid moiety, can be about 9 to about 12. Consideration should be given to the solubility and chemical compatibility of the HDAC inhibitor in choosing an appropriate excipient.

Subcutaneous formulations, preferably prepared according to procedures well known in the art at a pH in the range between about 5 and about 12, also include suitable buffers and isotonicity agents. They can be formulated to deliver a daily dose of HDAC inhibitor in one or more daily subcutaneous administrations, e.g., one, two or three times each day. The choice of appropriate buffer and pH of a formulation, depending on solubility of the HDAC inhibitor to be administered, is readily made by a person having ordinary skill in the art. Sodium chloride solution wherein the pH has been adjusted to the desired range with either acid or base, for example, hydrochloric acid or sodium hydroxide, can also be employed in the subcutaneous formulation. Typically, a pH range for the subcutaneous formulation can be in the range of from about 5 to about 12. A preferred pH range for subcutaneous formulation wherein the HDAC inhibitor has a hydroxamic acid moiety, can be about 9 to about 12. Consideration should be given to the solubility and chemical compatibility of the HDAC inhibitor in choosing an appropriate excipient.

The HDAC inhbitors can also be administered in intranasal form using suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regime.

The HDAC inhibitors can be administered as active ingredients in admixture with suitable pharmaceutical diluents, excipients or carriers (collectively referred to herein as "carrier" materials) suitably selected with respect to the intended form of administration, that is, oral tablets, capsules, elixers, syrups and the like, and consistent with conventional pharmaceutical practices.

For instance, for oral administration in the form of a tablet or capsule, the HDAC inhibitor can be combined with an oral, non-toxic, pharmaceutically acceptable, inert carrier such as lactose, starch, sucrose, glucose, methyl cellulose, microcrystalline cellulose, sodium croscarmellose, magnesium stearate, dicalcium phosphate, calcium sulfate, mannitol, sorbitol and the like or a combination thereof; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, com-sweeteners, natural and synthetic gums such as acacia, tragacanth or sodium alginate, carboxymethylcellulose, microcrystalline cellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch methyl cellulose, agar, bentonite, xanthan gum and the like.

Suitable pharmaceutically acceptable salts of the histone deacetylase compounds described herein and suitable for use in the method of the invention, are conventional non-toxic salts and can include a salt with a base or an acid addition salt such as a salt with an inorganic base, for example, an alkali metal salt (e.g. lithium salt, sodium salt, potassium salt, etc.), an alkaline earth metal salt (e.g. calcium salt, magnesium salt, etc.), an ammonium salt; a salt with an organic base, for example, an organic amine salt (e.g. triethylamine salt, pyridine salt, picoline salt, ethanolamine salt, triethanolamine salt, dicyclohexylamine salt, N,N'-dibenzylethylenediamine salt, etc.) etc.; an inorganic acid addition salt (e.g. hydrochloride, hydrobromide, sulfate, phosphate, etc.); an organic carboxylic or sulfonic acid addition salt (e.g. formate, acetate, trifluoroacetate, maleate, tartrate, methanesulfonate, benzenesulfonate, p-toluenesulfonate, etc.); a salt with a basic or acidic amino acid (e.g. arginine, aspartic acid, glutamic acid, etc.) and the like.

It is understood that standard therapy agents for the disease being treated, can be administered in combination with the HDAC inhibitors suitable for use in the invention. These standard agents can be administered by the methods described above for the HDAC inhibitors. The combination of agents, however, can be administered using the same or different methods.

### EXPERIMENTAL METHODS

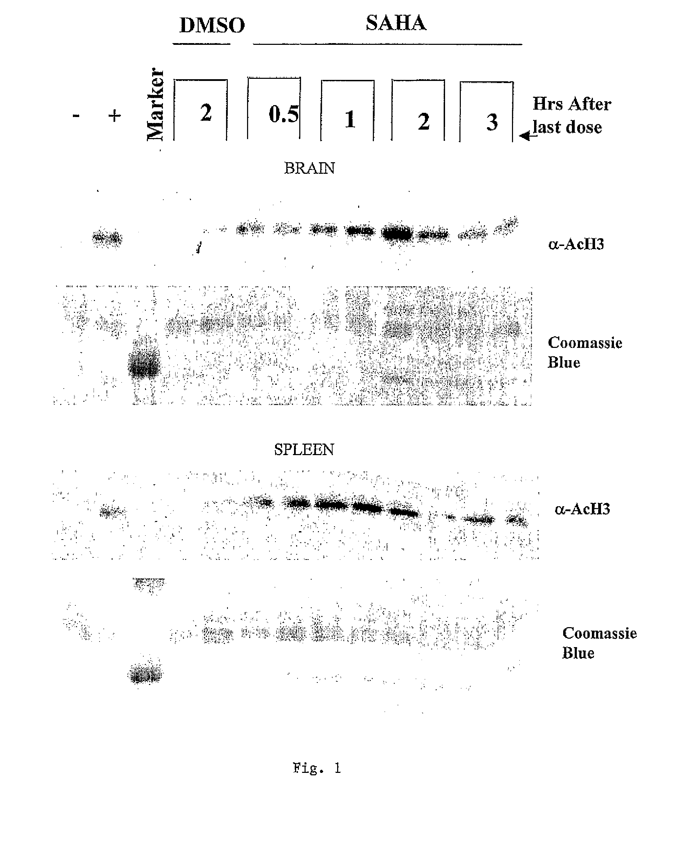

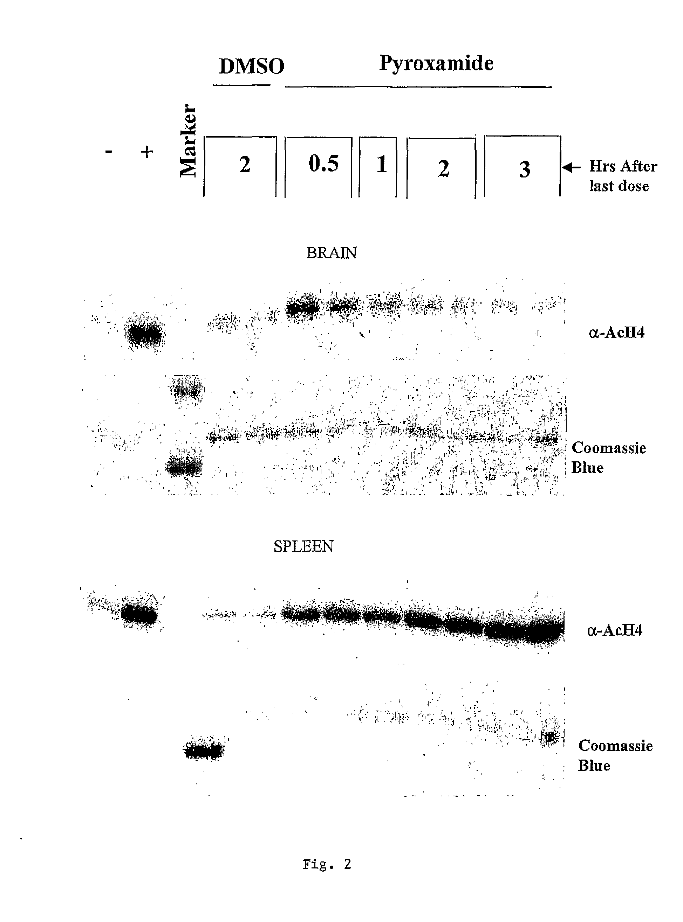

Mice (2 mice per condition) were injected by intraperitoneal injection (IP) with either SAHA (100 mg/kg), pyroxamide (200 mg/kg), or vehicle (dimethylsulfoxide). Each mouse was administered three injections at the indicated dose at 1 hour intervals. After the final IP injection, tissues (brain, spleen or liver) were isolated at the times indicated. Histones were isolated from tissues essentially as described by Yoshida et al., (1990) J. Biol. Chem. 265:17174-17179. Equal amounts of histones (1 µg) were electrophoresed on 15% SDS-polyacrylamide gels and transferred to Hybond-P filters (Amersham). Filters were blocked with 3% milk and probed with a rabbit purified polyclonal anti-acetylated histone H4 antibody (αAc-H4) and anti-acetylated histone H3 antibody (αAc-H3) (Upstate Biotechnology, Inc.). Levels of acetylated histone were visualized using a horseradish peroxidase-conjugated goat anti-rabbit antibody (1:5000) and the SuperSignal chemiluminescent substrate (Pierce). As a loading control for the histone proteins, parallel gels were run and stained with Coomassie Blue (CB). The results are shown in FIGS. 1 and 2.

All references cited herein are incorporated by reference in their entirety. While this invention has been particularly shown and described with references to preferred embodiments thereof it will be understood by those skilled in the art that various changes in form and details maybe made therein without departing from the meaning of the invention described.
1. A method of inhibiting histone deacetylase in the brain of a mammal comprising administering to the mammal a histone deacetylase inhibiting amount of a histone deacetylase inhibitor compound.
2. The method of Clause 1, wherein the histone deacetylase inhibitor compound is selected from: 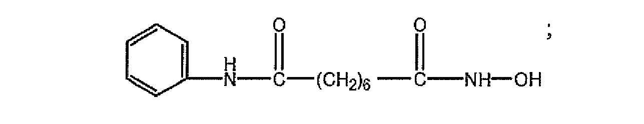 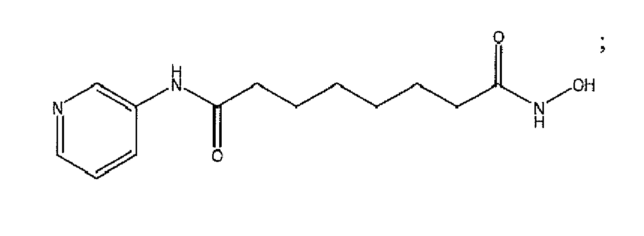 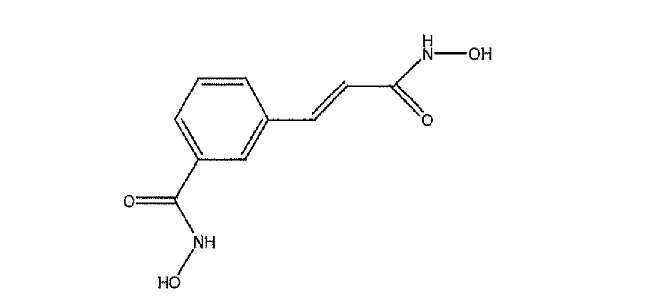 or a pharmaceutically acceptable salt thereof
3. The method of Clause 1, wherein the histone deacetylase inhibitor compound is represented by Formula II: 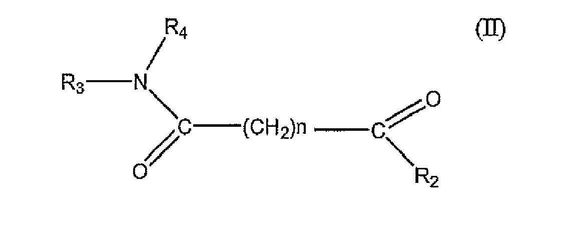 wherein each of R₃ and R₄ are independently the same as or different from each other and are a hydrogen atom, a hydroxyl group, a substituted or unsubstituted, branched or unbranched alkyl, alkenyl, cycloalkyl, arylalkyloxy, aryloxy, arylalkyloxy or pyridine group, or R₃ and R₄ bonded together to form a piperidine group, R₂ is a hydroxylamino, hydroxyl, amino, alkylamino, dialkylamino or alkyloxy group and n is an integer from about 4 to about 8 or a pharmaceutically acceptable salt thereof.
4. The method of Clause 1, wherein the histone deacetylase inhibitor compound is represented by Formula III: 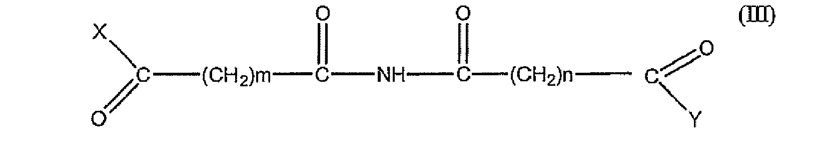 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino, or axyloxyalkylamino group; R is a hydrogen atom, a hydroxyl, group, a substituted or unsubstituted alkyl, arylakyloxy, or aryloxy group; and each of m and n are independently the same as or different from each other and are each an integer from about 0 to about 8 or a pharmaceutically acceptable salt thereof.
5. The method of Clause 1, wherein the histone deacetylase inhibitor compound is represented by structural Formula IV: 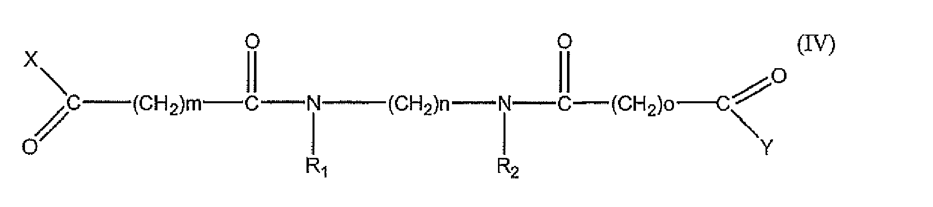 wherein each of X and Y are independently the same as or different from each other and are a hydroxyl, amino or hydroxylamino group, a substituted or unsbustituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m, n and o are independently the same as or different from each other and are each an integer from about 0 to about 8 or a pharmaceutically acceptable salt thereof.
6. The method of Clause 1, wherein the histone deacetylase inhibitor compound is represented by structural Formula V: 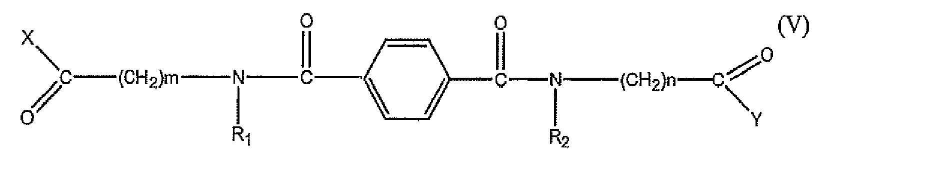 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy, group; and each ofm and n are independently the same as or different from each other and are each an integer from about 0 to about 8 or a pharmaceutically acceptable salt thereof.
7. The method of Clause 1, wherein the histone deacetylase inhibitor is represented by structural Formula (XIII): 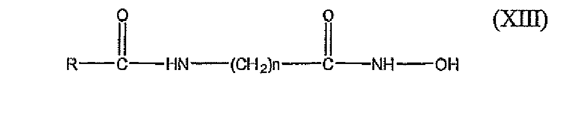 wherein R is a substituted or unsbustituted phenyl, piperidine, thiazole, 2-pyridine, 3-pyridine or 4-pyridine and n is an integer from about 4 to about 8 or a pharmaceutically acceptable salt thereof.
8. The method of Clause 1, wherein the histone deacetylase inhibitor compound is represented by structure Formula XVII: 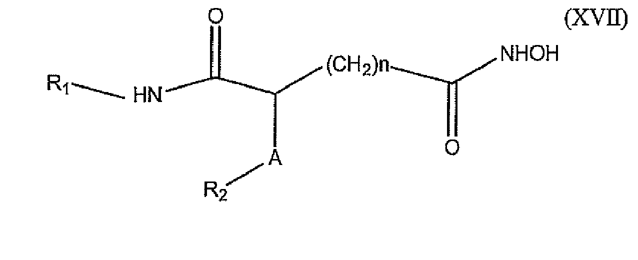 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine and n is an integer from 3 to 10 or a pharmaceutically acceptable salt thereof.
9. The method of Clause 8, wherein the histone deacetylase inhibitor compound has the following structure: 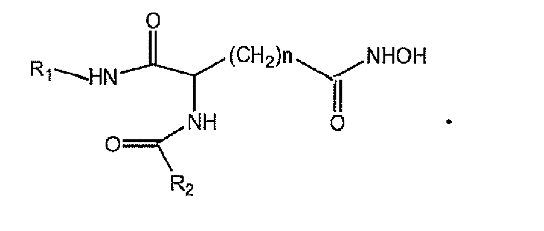
10. The method of Clause 9, wherein the histone deacetylase inhibitor compound has the formula: 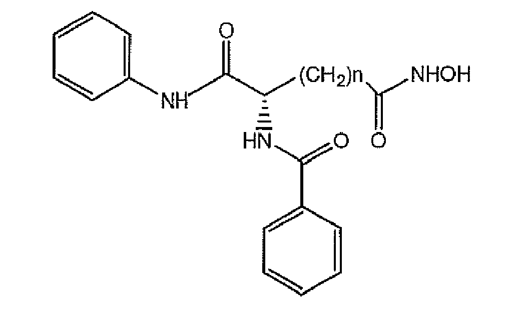 or a pharmaceutically acceptable salt thereof.
11. The method of Clause 9, wherein the histone deacetylase inhibitor compound has the formula: 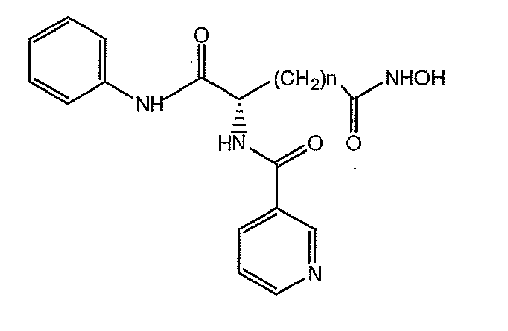 or a pharmaceutically acceptable salt thereof.
12. The method of Clause 1, wherein the histone deacetylase inhibitor compound is represented by structural Formula XXII: 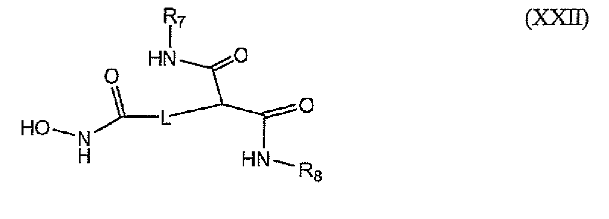 wherein L is a linker selected from the group consisting of -(CH₂)ₙ-, -(CH=CH)ₘ-, -phenyl-, -cycloalkyl-, or any combination thereof; and
   wherein each of R₇ and R₈ are independently substituted or unsubstituted, aryl, naphtha, pyridineamino, 9-purine-6-amine, thiazoleamino group, aryloxy, arylalkyloxy, or pyridine group, n is an integer from 3 to 10 and m is an integer from 0-10.
13. The method of Clause 12, wherein the histone deacetylase inhibitor compound has the structure: 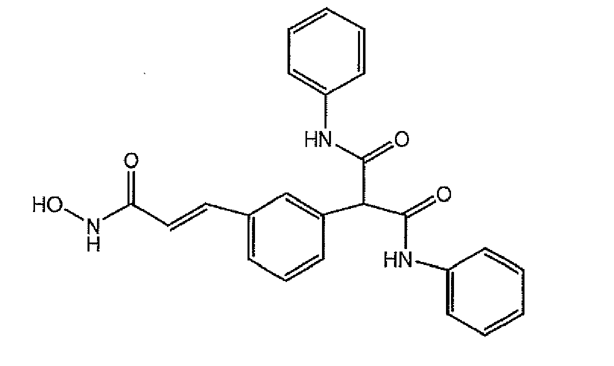 or a pharmaceutically acceptable salt thereof.
14. The method of Clause 1, wherein the histone deacetylase inhibitor compound is represented by structural Formula XXI: 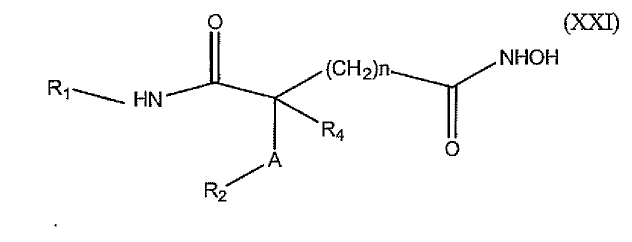 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine, R₄ is hydrogen, a halogen, a phenyl or a cycloalkyl moiety and n is an integer from 3 to 10 or a pharmaceutically acceptable salt thereof.
15. The method of Clause 14, wherein the histone deacetylase inhibitor compound has the structure: 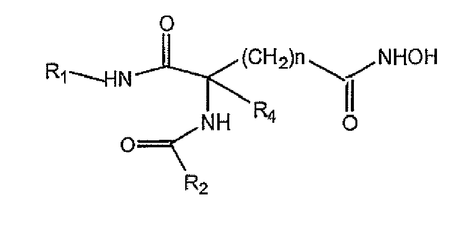 or a phamiaceutically acceptable salt thereof.
16. The method of Clause 14, wherein the histone deacetylase inhibitor compound has the structure: 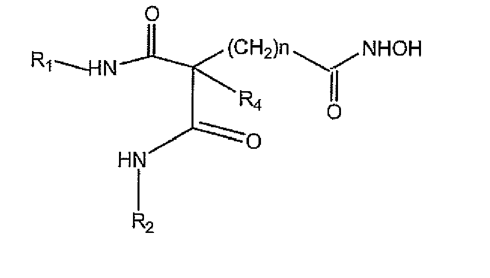 or a pharmaceutically acceptable salt thereof:
17. The method of Clause 1, wherein the histone deacetylase inhibitor compound is represented by structural Formula XVIII: 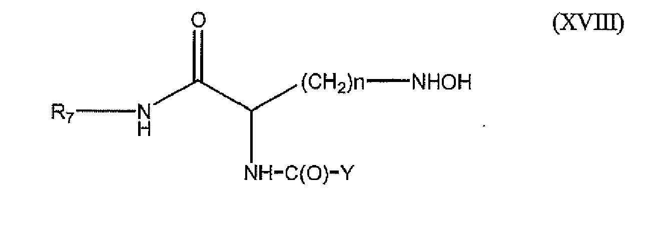 wherein R₇ is selected from substituted or unsubstituted aryl (e.g., phenyl or naphthyl), pyridineamino, 9-purine-6-amine, thiazoleamino, aryloxy, arylalkyloxy or pyridine and n is an integer from 3 to 10 and Y is selected from 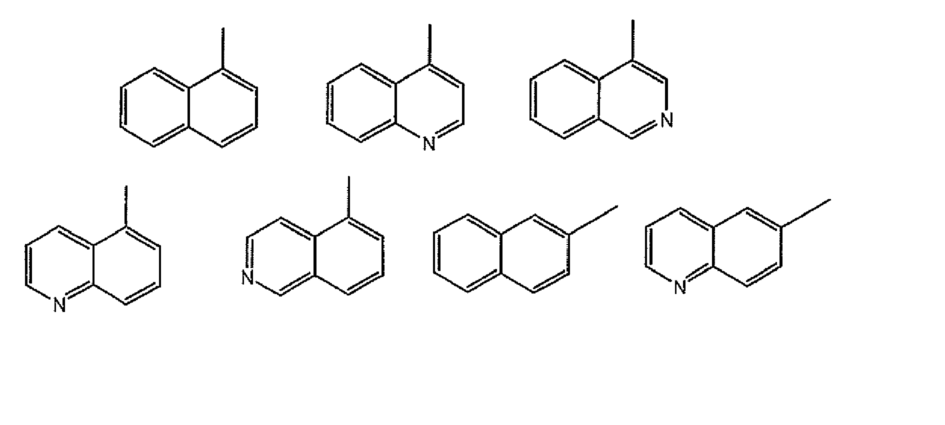 or a pharmaceutically acceptable salt thereof.
18. The method of Clause 1, wherein the histone deacetylase inhibitor compound is represented by structural Formula XIX: 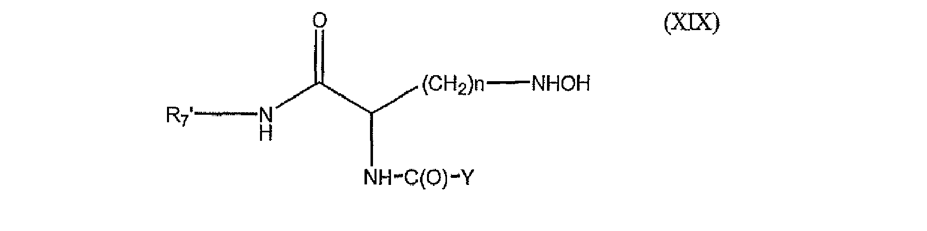 wherein n is an integer from 3 to 10, Y is selected from 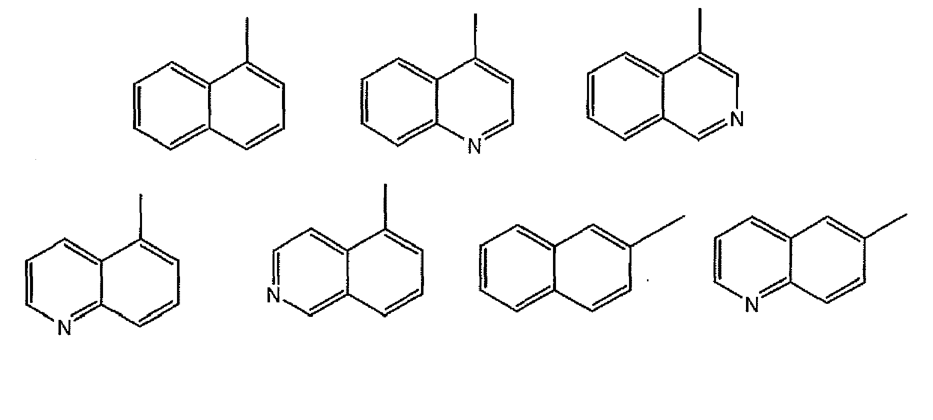 R₇' is selected from 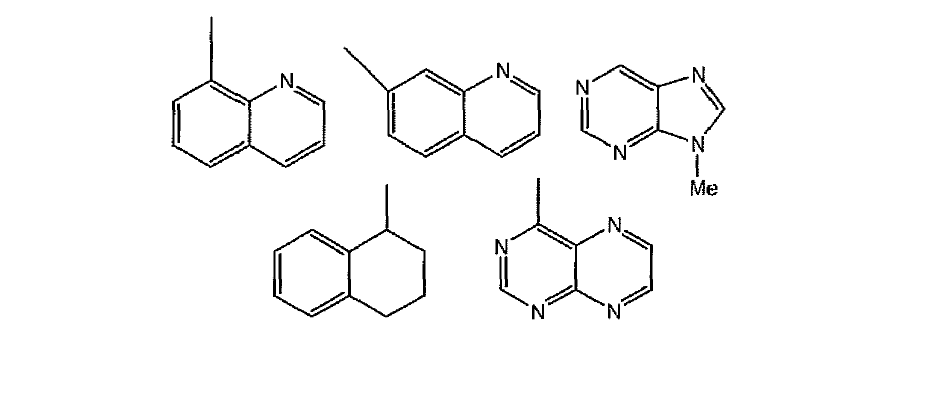 or a phannaccutically acceptable salt thereof.
19. The method of Clause 1, wherein the histone deacetylase inhibiting compound is represented by structural Formula XX: 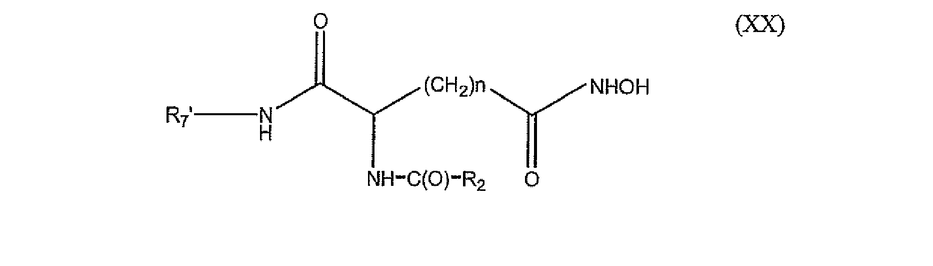 wherein R₂ is selected from substituted or unsubstituted aryl, substituted or unsubstituted naphtha, pyridineamino, 9-purine-6-amine, thiazoleamino, substituted or unsubstituted aryloxy, substituted or unsubstituted arylalkyloxy or pyridine and n is an integer from 3 to 10 and R₇' is selected from 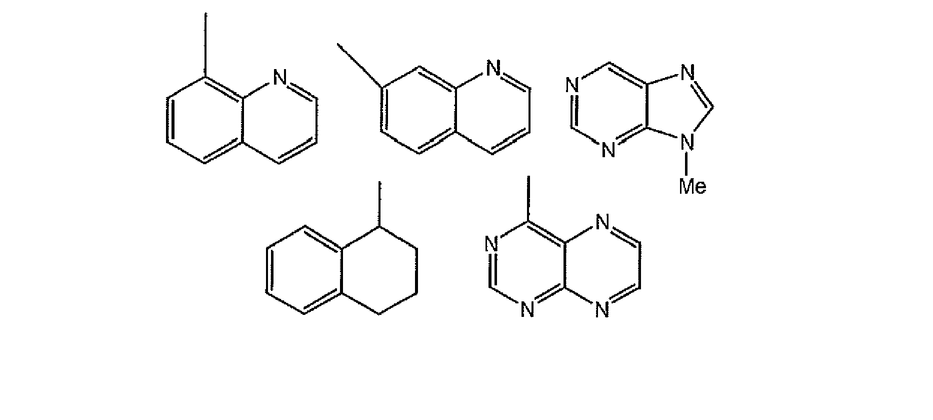 or a pharmaceutically acceptable salt thereof.
20. The method of Clause 1, wherein the histone deacetylase inhibitor compound has the formula: 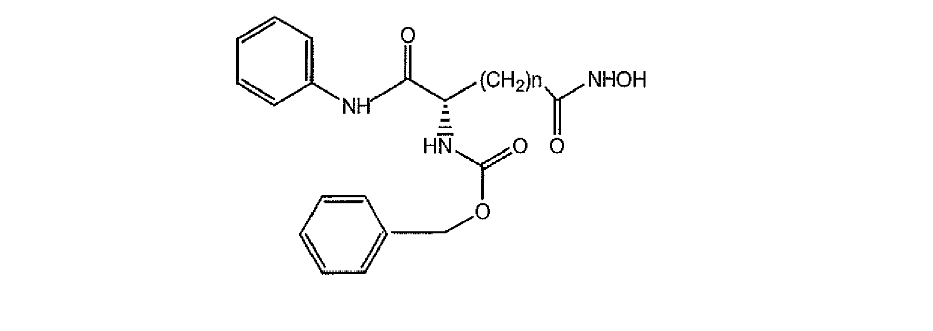 wherein n is an integer from 3 to 10 or a pharmaceutically acceptable salt thereof.
21. The method of Clause 1, wherein the histone deacetylase inhibiting compound has the formula: 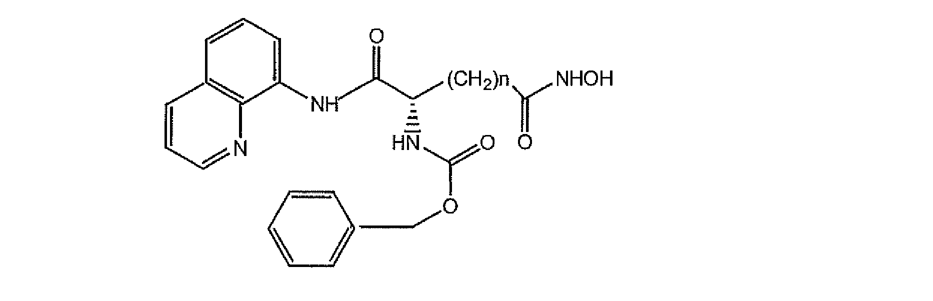 wherein n is an integer from 3 to 10 or a pharmaceutically acceptable salt thereof.
22. The method of Clause 1, wherein the histone deacetylase inhibiting compound has the formula: 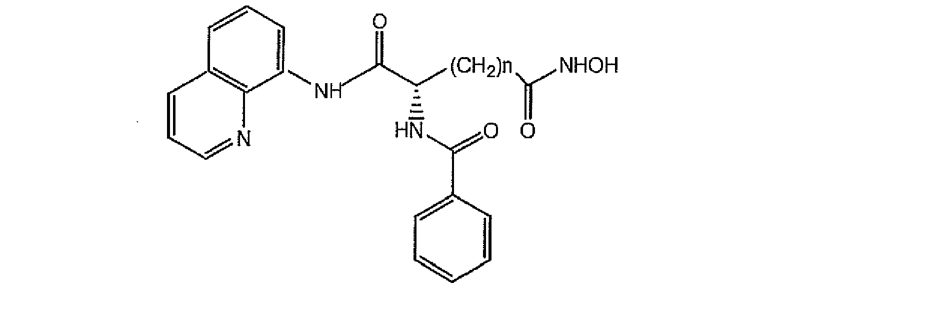 wherein n is an integer from 3 to 10 or a pharmaceutically acceptable salt thereof.
23. The method of Clause 1, wherein the histone deacetylase inhibiting compound has the formula: 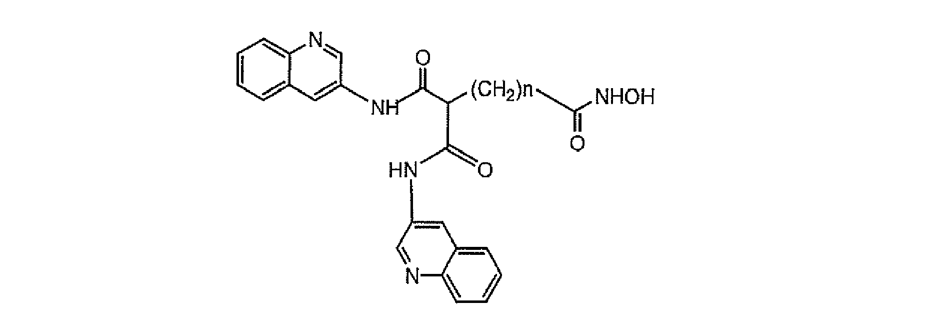 wherein n is an integer from 3 to 10 or a pharmaceutically acceptable salt thereof.
24. The method of Clause 1, wherein the histone deacetylase inhibiting compound has the formula: 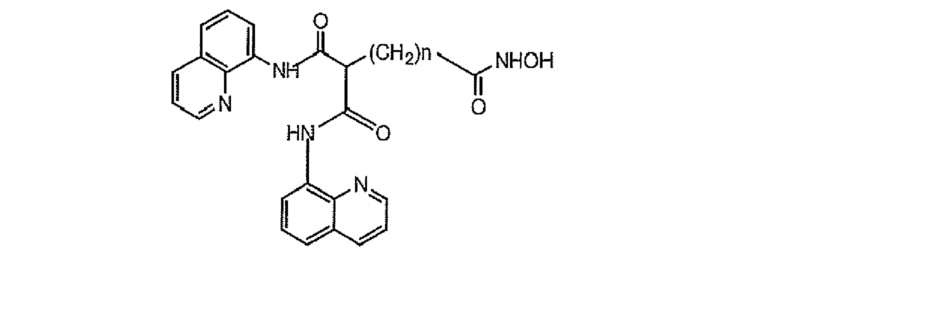 wherein n is an integer from 3 to 10 or a pharmaceutically acceptable salt thereof.
25. The method of Clause 1, wherein the histone deacetylase inhibiting compound has the formula: 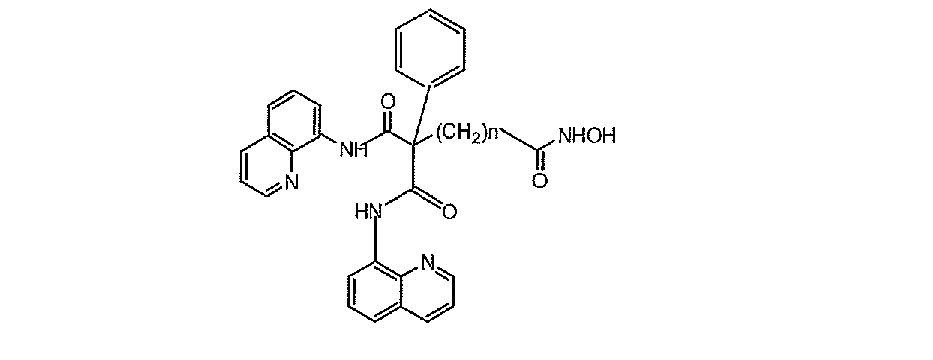 wherein n is an integer from 3 to 10 or a pharmaceutically acceptable salt thereof.
26. The method of Clause 1, wherein the histone deacetylase inhibiting compound has the formula: 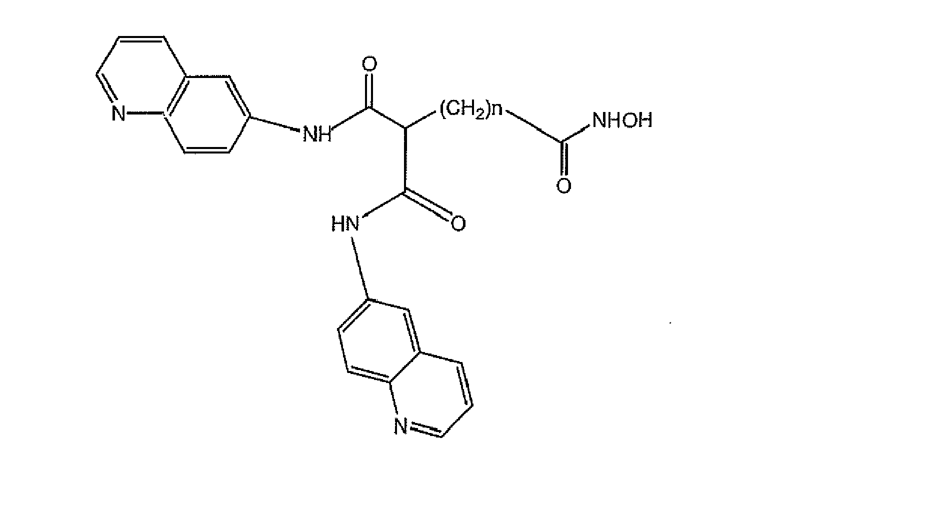 wherein n is an integer from 3 to 10 or a pharmaceutically acceptable salt thereof.
27. The method of Clause 1, wherein the histone deacetylase inhibiting compound has the formula: 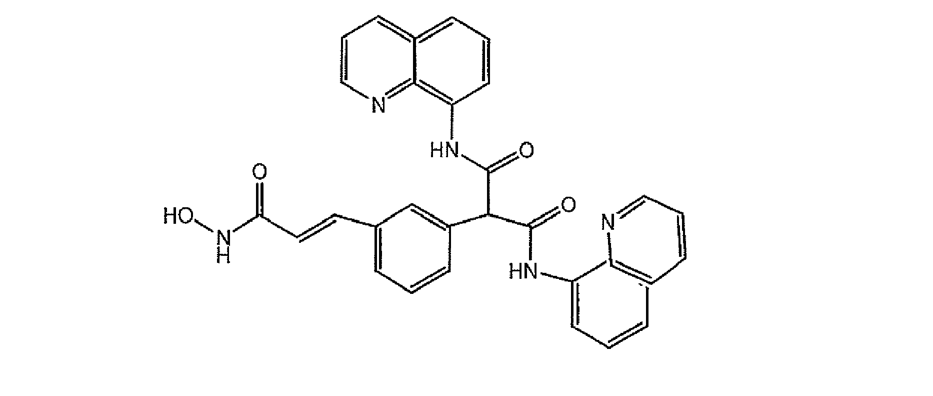 or a pharmaceutically acceptable salt thereof.
28. A method of treating a disease of the central nervous system in an mammal in need thereof comprising administering to the mammal a therapeutically effective amount of a histone deacetylase inhibitor compound.
29. The method of Clause 28 wherein the disease is a polyglutamine expansion disease.
30. The method of Clause 29 wherein the polyglutamine expansion disease is Huntington's disease.
31. The method of Clause 28 wherein the mammal is a human.
32. The method of Clause 28 wherein the inhibitor of histone deacetylase is selected from: 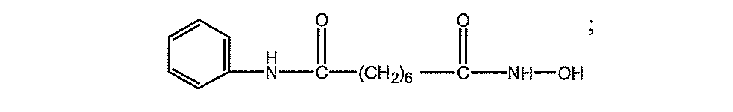 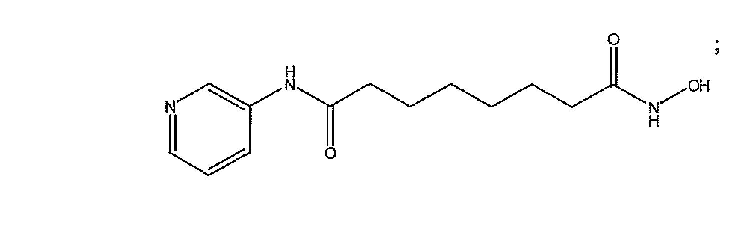 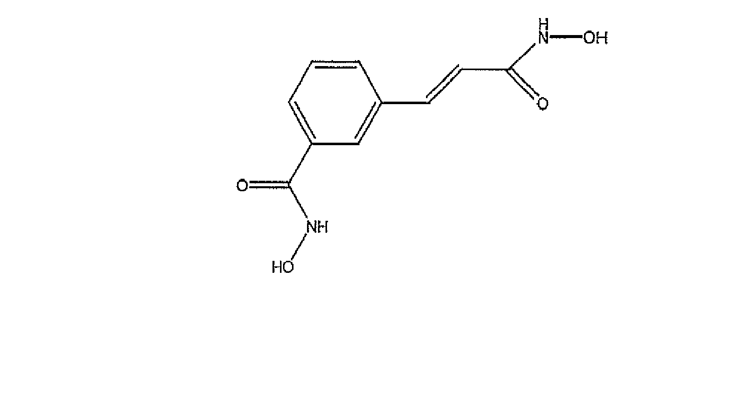 or a pharmaceutically acceptable salt thereof.
33. The method of Clause 28, wherein the histone deacetylase inhibitor compound is represented by Formula II: 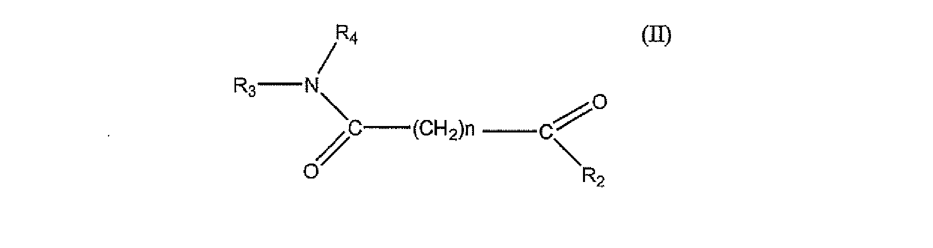 wherein each of R₃ and R₄ are independently the same as or different from each other and are a hydrogen atom, a hydroxyl group, a substituted or unsubstituted, branched or unbranched alkyl, alkenyl, cycloalkyl, arylalkyloxy, aryloxy, arylalkyloxy or pyridine group, or R₃ and R₄ bonded together to form a piperidine group, R₂ is a hydroxylamino, hydroxyl, amino, alkylamino, dialkylamino or alkyloxy group and n is an integer from about 4 to about 8 or a pharmaceutically acceptable salt thereof.
34. The method of Clause 28, wherein the histone deacetylase inhibitor compound is represented by Formula III: 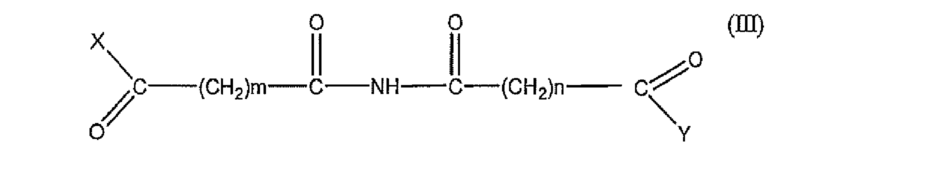 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino, or aryloxyakylamino group; R is a hydrogen atom, a hydroxyl, group, a substituted or unsubstituted alkyl, arylalkyloxy, or aryloxy group; and each of m and n are independently the same as or different from each other and are each an integer from about 0 to about 8 or a pharmaceutically acceptable salt thereof.
35. The method of Clause 28, wherein the histone deacetylase inhibitor compound is represented by structural Formula IV: 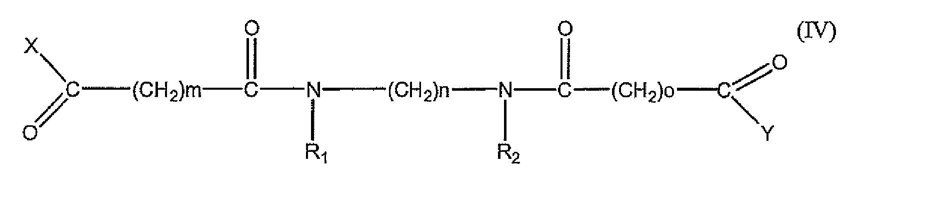 wherein each of X and Y are independently the same as or different from each other and are a hydroxyl, amino or hydroxylamino group, a substituted or unsbustituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m, n and o are independently the same as or different from each other and are each an integer from about 0 to about 8 or a pharmaceutically acceptable salt thereof.
36. The method of Clause 28, wherein the histone deacetylase inhibitor compound is represented by structural Formula V: 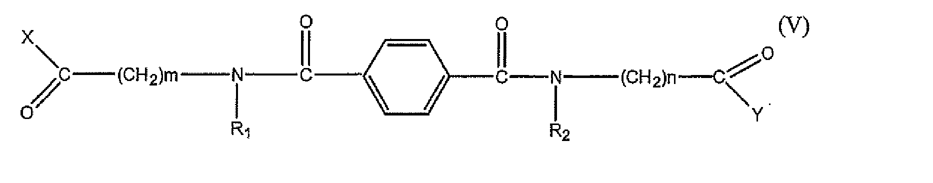 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m and n are independently the same as or different from each other and are each an integer from about 0 to about 8 or a pharmaceutically acceptable salt thereof.
37. The method of Clause 28, wherein the histone deacetylase inhibitor is represented by structural Formula (XIII): 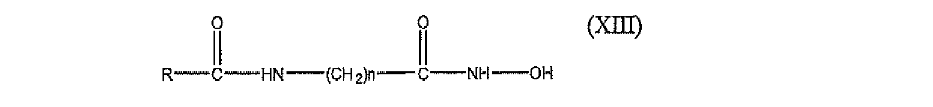 wherein R is a substituted or unsbustituted phenyl, piperidine, thiazole, 2-pyridine, 3-pyridine or 4-pyridine and n is an integer from about 4 to about 8 or a pharmaceutically acceptable salt thereof.
38. The method of Clause 28, wherein the histone deacetylase inhibitor compound is represented by structure Formula XVII: 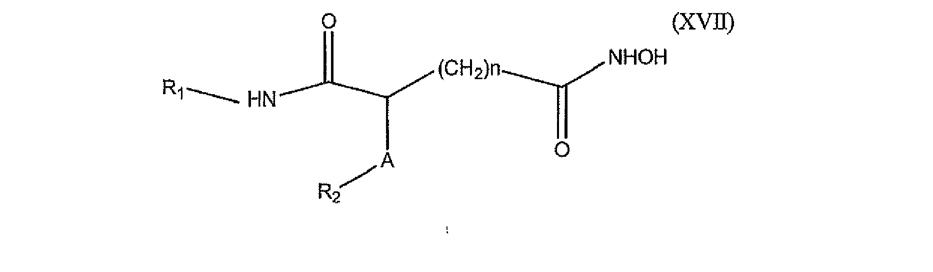 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine and n is an integer from 3 to 10 or a pharmaceutically acceptable salt thereof
39. The method of Clause 38, wherein the histone deacetylase inhibitor compound has the following structure: 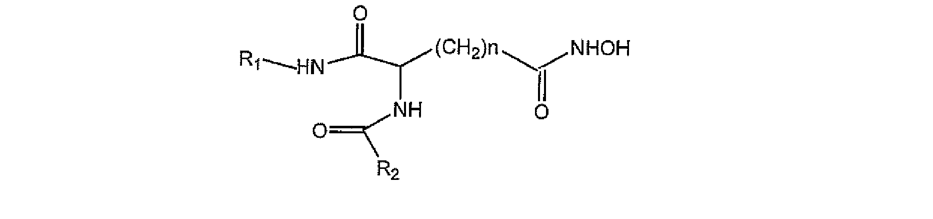
40. The method of Clause 39, wherein the histone deacetylase inhibitor compound has the formula: 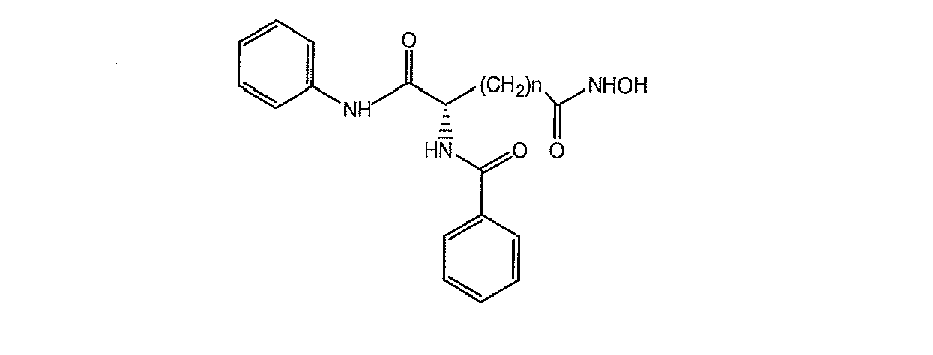 or a pharmaceutically acceptable salt thereof.
41. The method of Clause 39, wherein the histone deacetylase inhibitor compound has the formula: 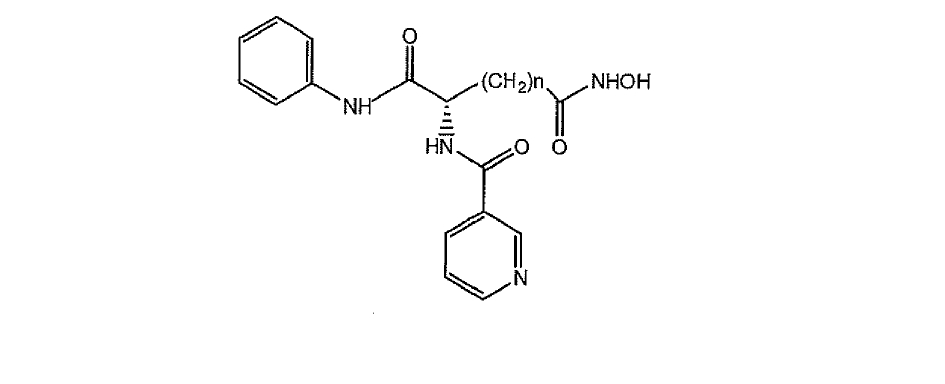 or a pharmaceutically acceptable salt thereof.
42. The method of Clause 28, wherein the histone deacetylase inhibitor compound is represented by structural Formula XXII: 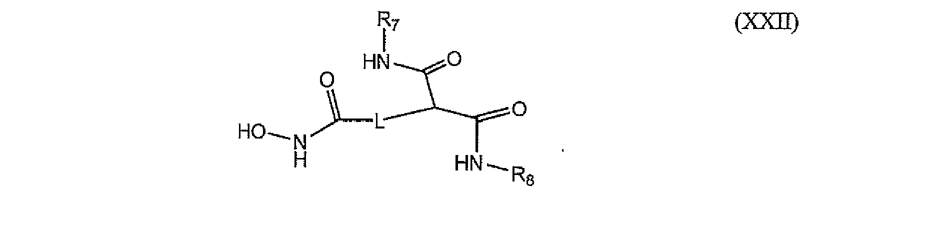 wherein L is a linker selected from the group consisting of -(CH₂)ₙ-, -(CH=CH)ₘ-, -phenyl-, -cycloalkyl-, or any combination thereof; and
   wherein each of R₇ and R₈ are independently substituted or unsubstituted, aryl, naphtha, pyridineamino, 9-purme-6-amine, thiazoleamino group, aryloxy, arylalkyloxy, or pyridine group, n is an integer from 3 to 10 and m is an integer from 0-10.
43. The method of Clause 42, wherein the histone deacetylase inhibitor compound has the structure: 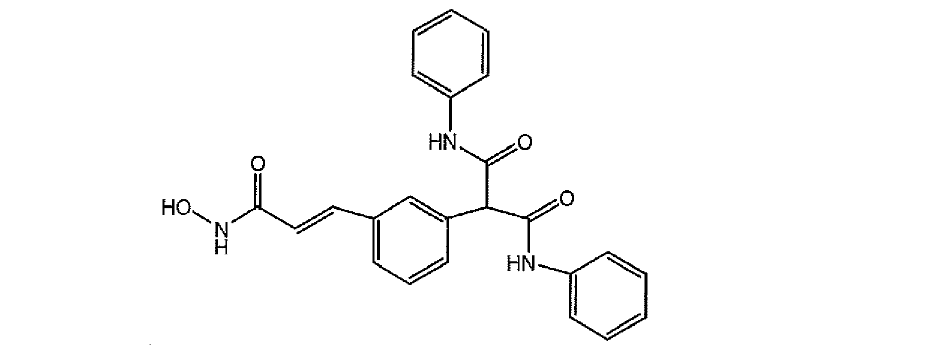 or a pharmaceutically acceptable salt thereof.
44. The method of Clause 28, wherein the histone deacetylase inhibitor compound is represented by structural Formula XXI: 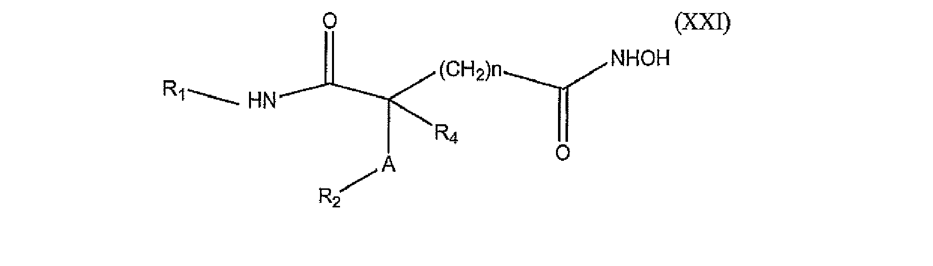 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine, R₄ is hydrogen, a halogen, a phenyl or a cycloalkyl moiety and n is an integer from 3 to 10 or a pharmaceutically acceptable salt thereof.
45. The method of Clause 44, wherein the histone deacetylase inhibitor compound has the structure: 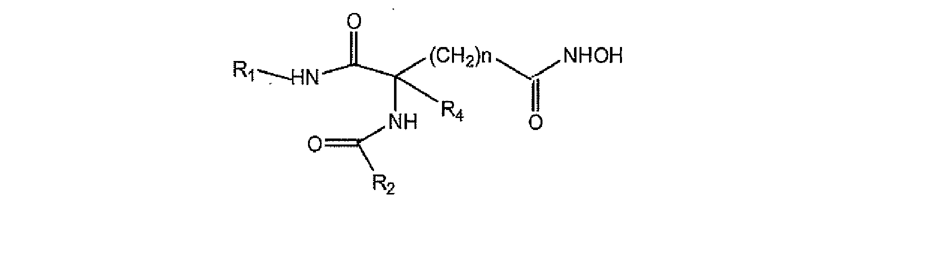 or a pharmaceutically acceptable salt thereof
46. The method of Clause 44, wherein the histone deacetylase inhibitor compound has the structure: 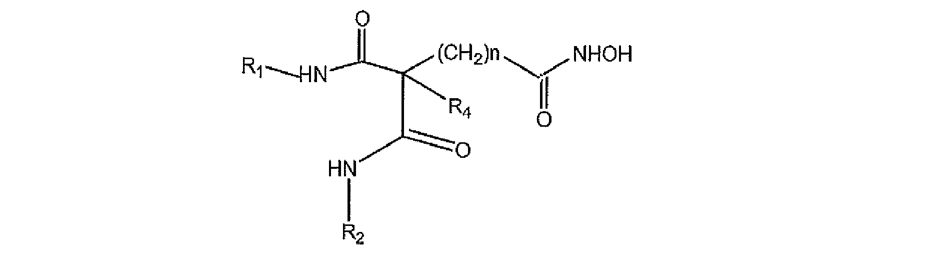 or a pharmaceutically acceptable salt thereof.
47. The method of Clause 28, wherein the histone deacetylase inhibitor compound is represented by structural Formula XVIII: 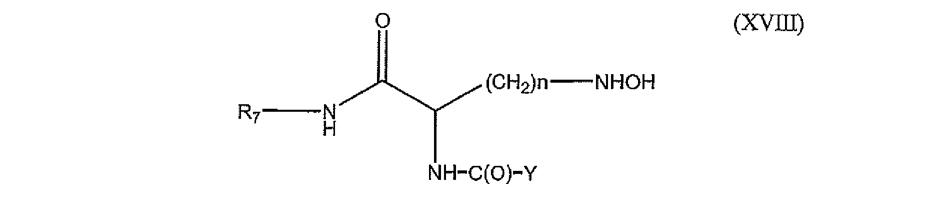 wherein R₇ is selected from substituted or unsubstituted aryl (e.g., phenyl or naphthyl), pyridineamino, 9-purine-6-amine, thiazoleamino, aryloxy, arylalkyloxy or pyridine and n is an integer from 3 to 10 and Y is selected from 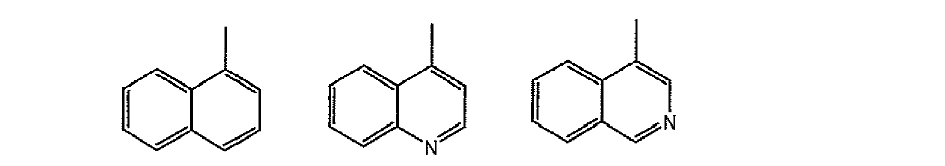 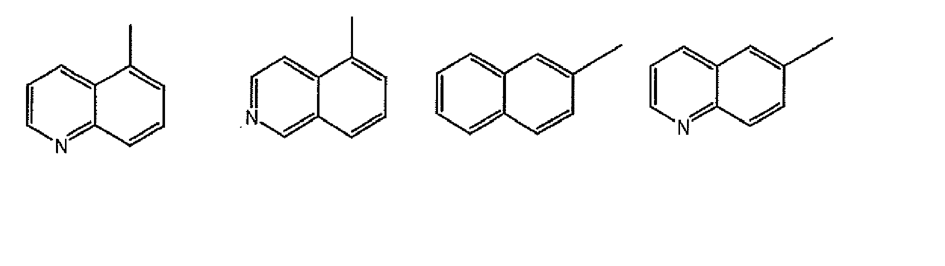 or a pharmaceutically acceptable salt thereof.
48. The method of Clause 28, wherein the histone deacetylase inhibitor compound is represented by structural Formula XIX: 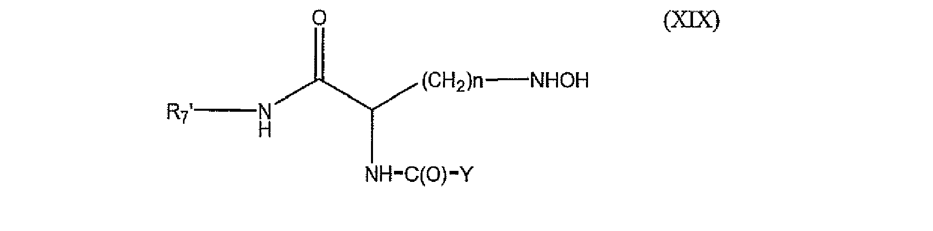 wherein n is an integer from 3 to 10, Y is selected from 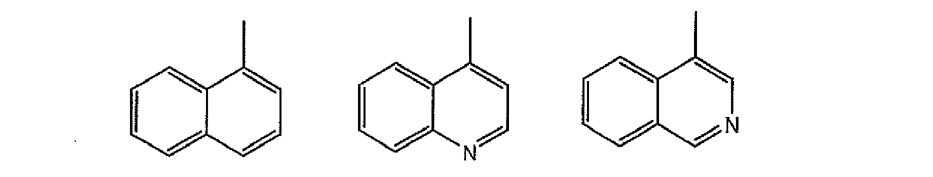 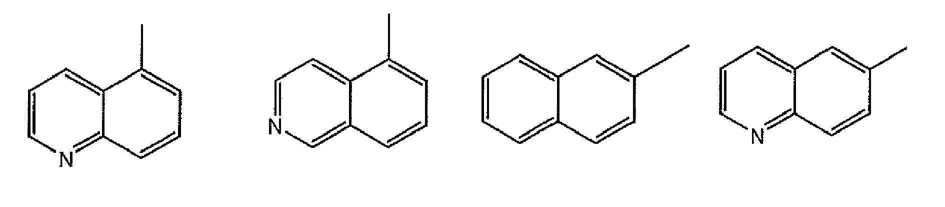 R₇' is selected from 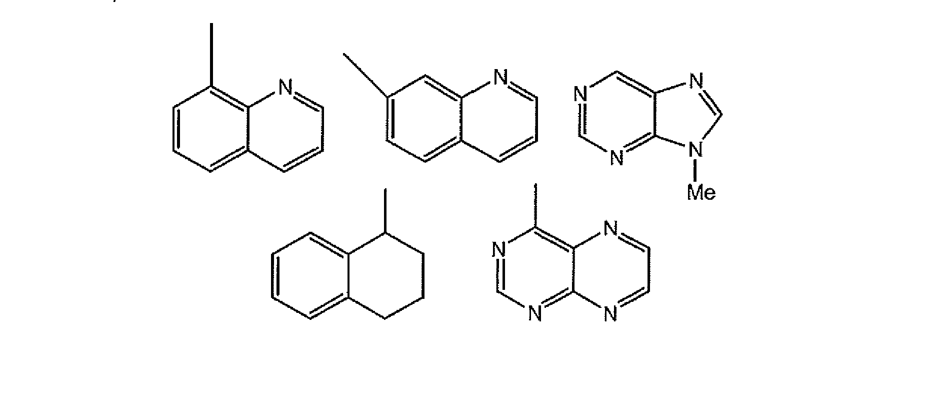 or a pharmaceutically acceptable salt thereof
49. The method of Clause 28, wherein the histone deacetylase inhibiting compound is represented by structural Formula XX: 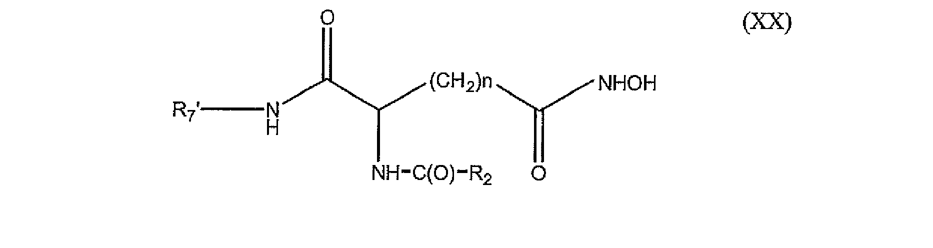 wherein R₂ is selected from substituted or unsubstituted aryl, substituted or unsubstituted naphtha, pyridineamino, 9-purine-6-amine, thiazoleamino, substituted or unsubstituted aryloxy, substituted or unsubstituted arylalkyloxy or pyridine and n is an integer from 3 to 10 and R₇' is selected from 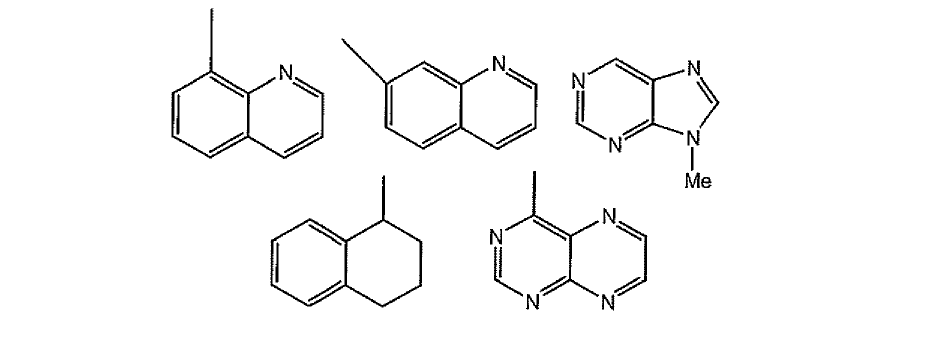 or a pharmaceutically acceptable salt thereof.
50. The method of Clause 28, wherein the histone deacetylase inhibitor compound has the formula: 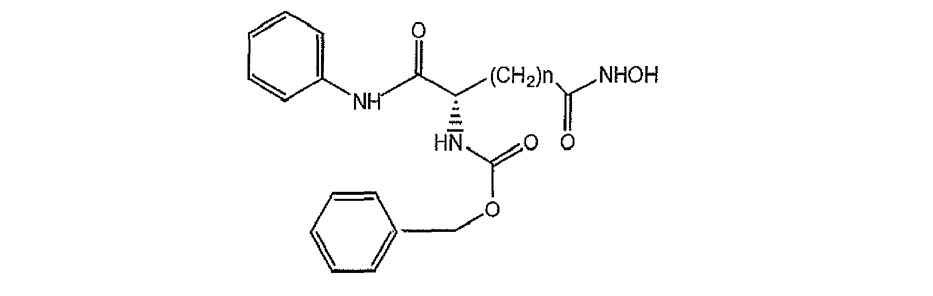 wherein n is an integer from 3 to 10 or a pharmaceuticaly acceptable salt thereof.
51. The method of Clause 28, wherein the histone deacetylase inhibiting compound has the formula: 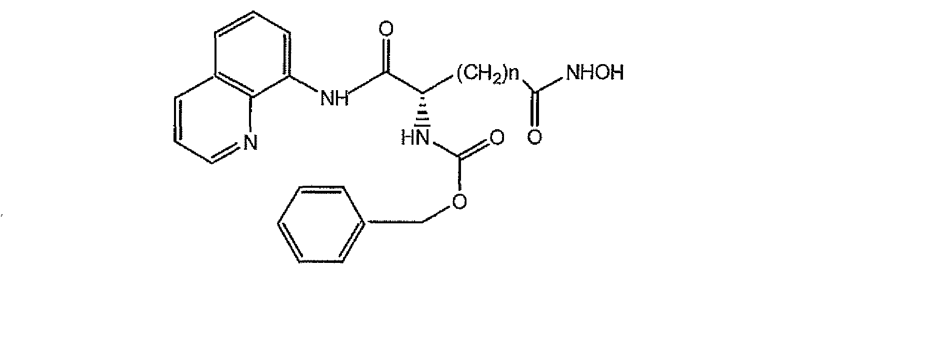 wherein n is an integer from 3 to 10 or a pharmaceutically acceptable salt thereof.
52. The method of Clause 28, wherein the histone deacetylase inhibiting compound has the formula: 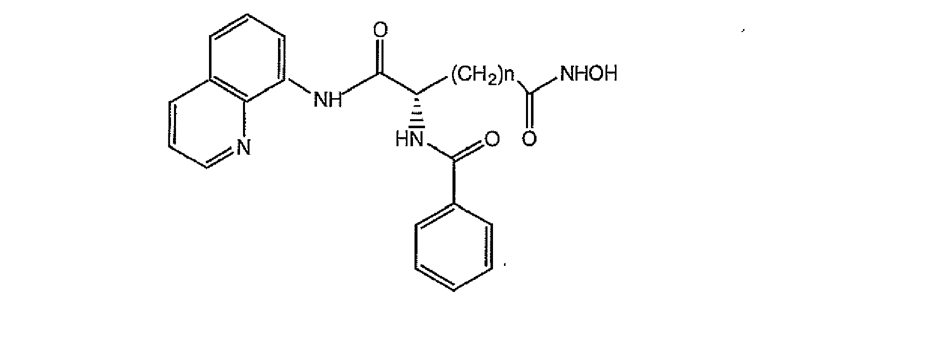 wherein n is an integer from 3 to 10 or a pharmaceutically acceptable salt thereof.
53. The method of Clause 28, wherein the histone deacetylase inhibiting compound has the formula: 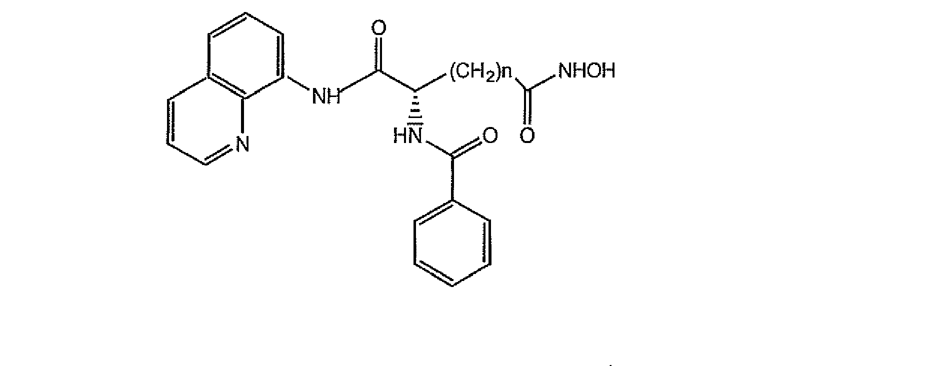 wherein n is an integer from 3 to 10 or a pharmaceutically acceptable salt thereof.
54. The method of Clause 28, wherein the histone deacetylase inhibiting compound has the formula: 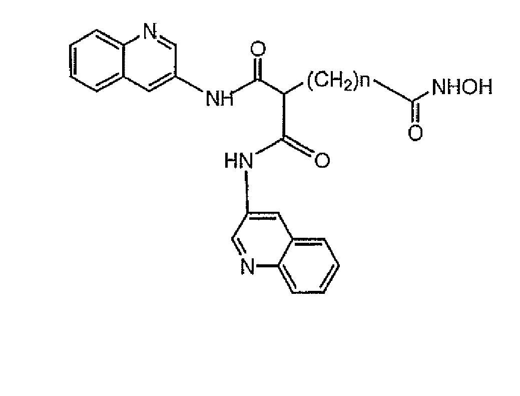 wherein n is an integer from 3 to 10 or a pharmaceutically acceptable salt thereof.
55. The method of Clause 28, wherein the histone deacetylase inhibiting compound has the formula: 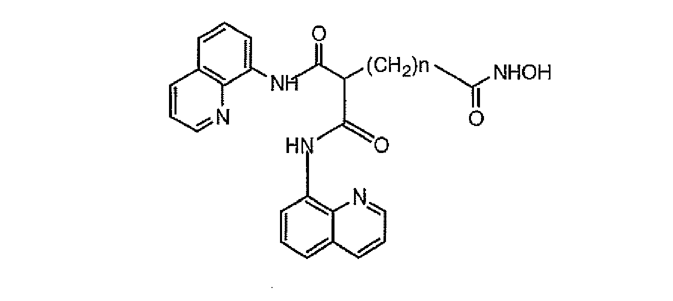 wherein n is an integer from 3 to 10 or a pharmaceutically acceptable salt thereof.
56. The method of Clause 28, wherein the histone deacetylase inhibiting compound has the formula: 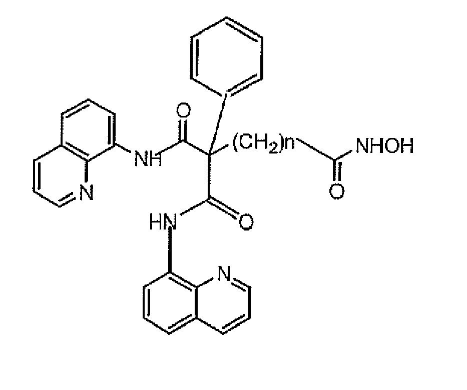 wherein n is an integer from 3 to 10 or a pharmaceutically acceptable salt thereof.
57. The method of Clause 28, wherein the histone deacetylase inhibiting compound has the formula: 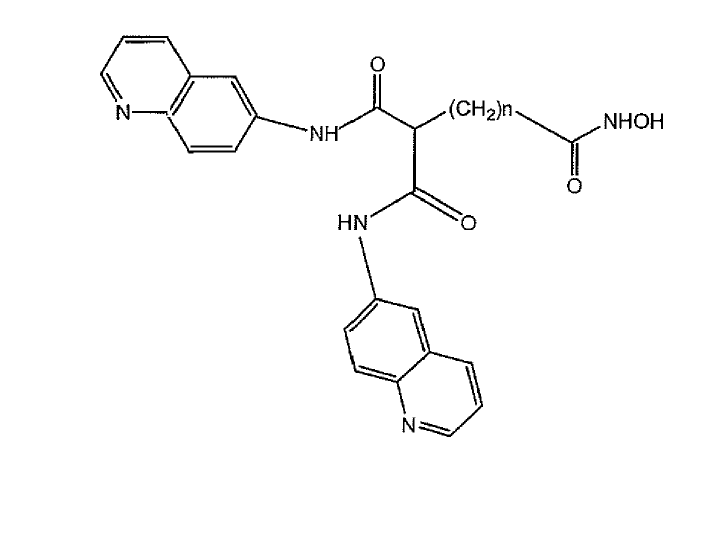 wherein n is an integer from 3 to 10 or a pharmaceutically acceptable salt thereof.
58. The method of Clause 28, wherein the histone deacetylase inhibiting compound has the formula: 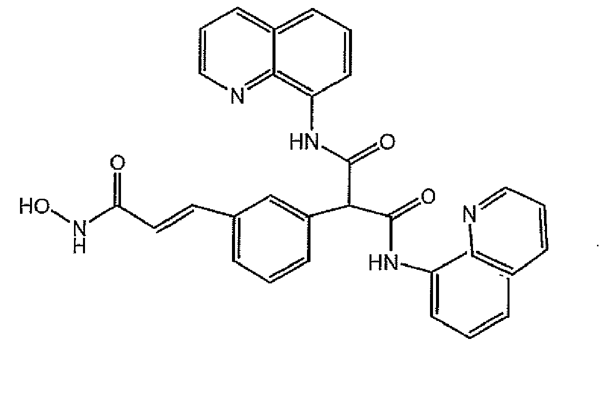 or a pharmaceutically acceptable salt thereof.
59. A method of treating brain cancer in a mammal in need thereof comprising administering to the mammal a therapeutically effective amount of a histone deacetylase inhibitor compound.
60. The method of Clause 59 wherein the histone deacetylase inhibitor compound is selected from: 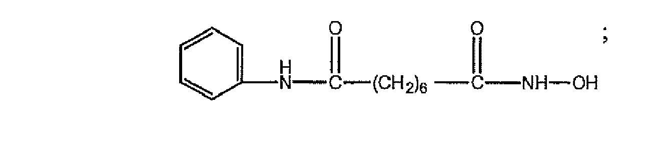 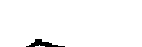 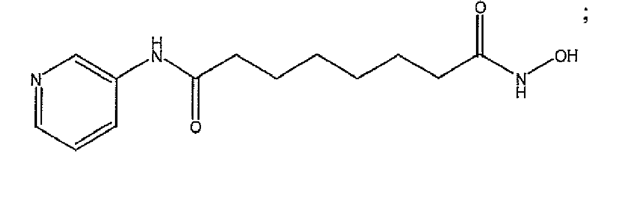 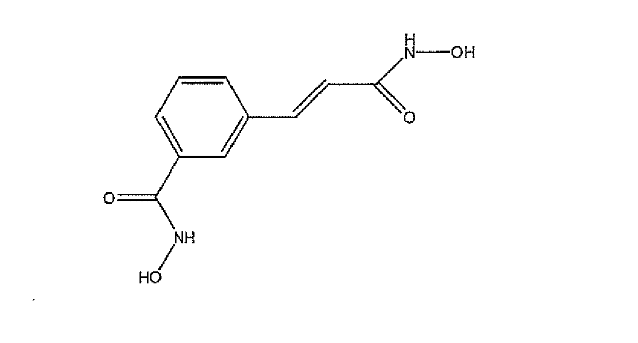 or a pharmaceutically acceptable salt thereof.
61. The method of Clause 59, wherein the histone deacetylase inhibitor compound is represented by Formula II: 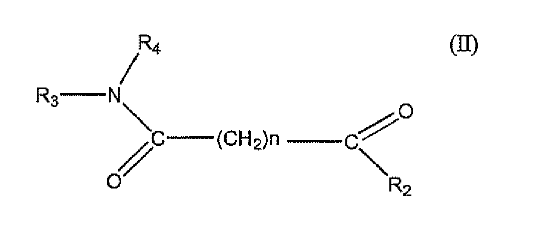 wherein each of R₃ and R₄ are independently the same as or different from each other and are a hydrogen atom, a hydroxyl group, a substituted or unsubstituted, branched or unbranched alkyl, alkenyl, cycloalkyl, arylalkyloxy, aryloxy, arylalkyloxy or pyridine group, or R₃ and R₄ bonded together to form a piperidine group, R₂ is a hydroxylamino, hydroxyl, amino, alkylamino, dialkylamino or alkyloxy group and n is an integer from about 4 to about 8 or a pharmaceutically acceptable salt thereof.
62. The method of Clause 59, wherein the histone deacetylase inhibitor compound is represented by Formula III: 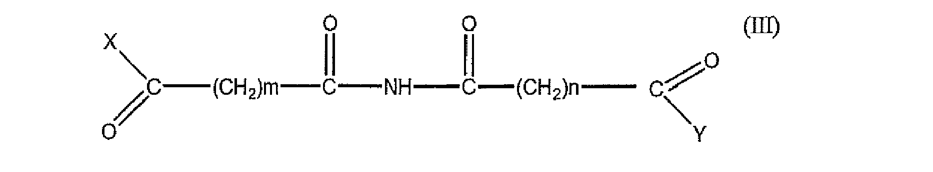 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino, or aryloxyalkylamino group; R is a hydrogen atom, a hydroxyl, group, a substituted or unsubstituted alkyl, arylalkyloxy, or aryloxy group; and each of m and n are independently the same as or different from each other and are each an integer from about 0 to about 8 or a pharmaceutically acceptable salt thereof.
63. The method of Clause 59, wherein the histone deacetylases inhibitor compound is represented by structural Formula IV: 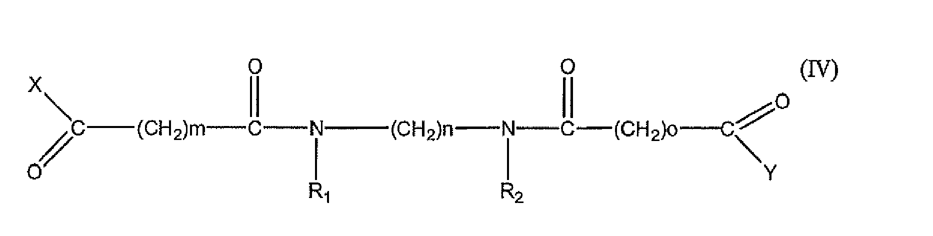 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m, n and o are independently the same as or different from each other and are each an integer from about 0 to about 8 or a pharmaceutically acceptable salt thereof
64. The method of Clause 59, wherein the histone deacetylase inhibitor compound is represented by structural Formula V: 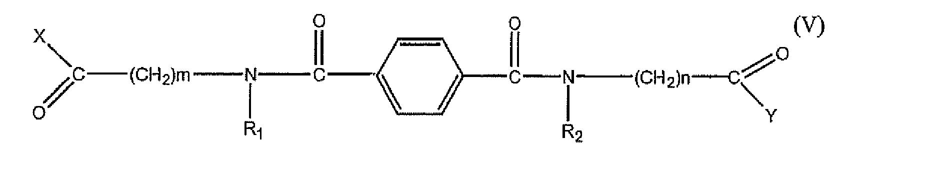 wherein each of X and Y are independently the same as or different from each other and are a hydroxyl, amino or hydroxylamino group, a substituted or unsubstituted alkyloxy, akylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m and n are independently the same as or different from each other and are each an integer from about 0 to about 8 or a pharmaceutically acceptable salt thereof.
65. The method of Clause 59, wherein the histone deacetylase inhibitor is represented by structural Formula (XIII): 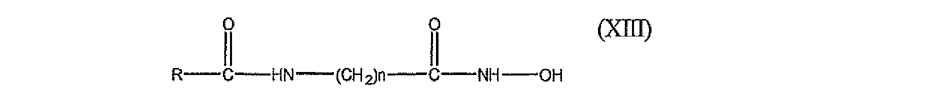 wherein R is a substituted or unsbustituted phenyl, piperidine, thiazole, 2-pyridine, 3-pyridine or 4-pyridine and n is an integer from about 4 to about 8 or a pharmaceutically acceptable salt thereof.
66. The method of Clause 59, wherein the histone deacetylase inhibitor compound is represented by structure Formula XVII: 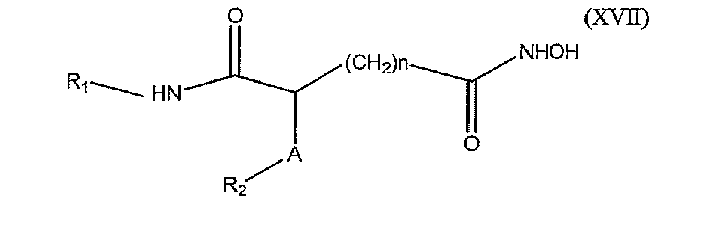 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine and n is an integer from 3 to 10 or a pharmaceutically acceptable salt thereof.
67. The method of Clause 66, wherein the histone deacetylase inhibitor compound has the following structure: 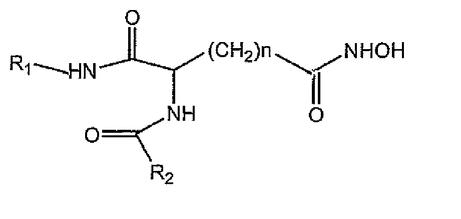
68. The method of Clause 67, wherein the histone deacetylase inhibitor compound has the formula: 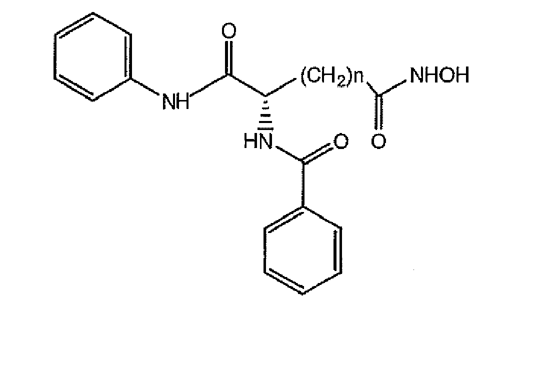 or a pharmaceutically acceptable salt thereof.
69. The method of Clause 67, wherein the histone deacetylase inhibitor compound has the formula: 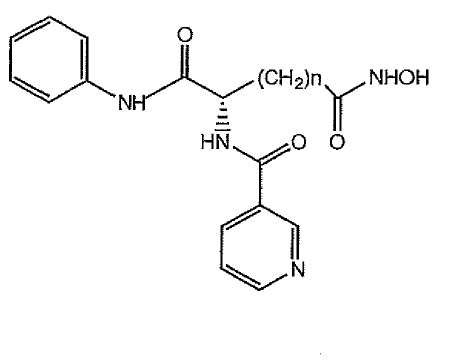 or a pharmaceutically acceptable salt thereof.
70. The method of Clause 59, wherein the histone deacetylase inhibitor compound is represented by structural Formula XXII: 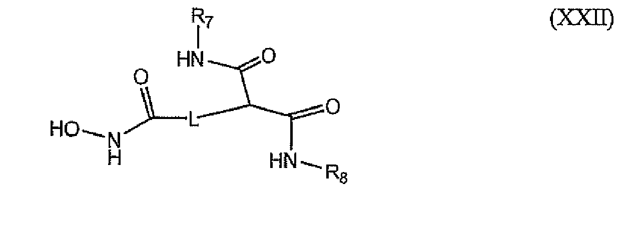 wherein L is a linker selected from the group consisting of -(CH₂)ₙ-, -(CH=CH)ₘ-, -phenyl-, -cycloalkyl-, or any combination thereof; and
   wherein each of R₇ and R₈ are independently substituted or unsubstituted, aryl, naphtha, pyridineamino, 9-purine-5-amine, thiazoleamino group, aryloxy, arylalkyloxy, or pyridine group, n is an integer from 3 to 10 and m is an integer from 0-10.
71. The method of Clause 70, wherein the histone deacetylase inhibitor compound has the structure: 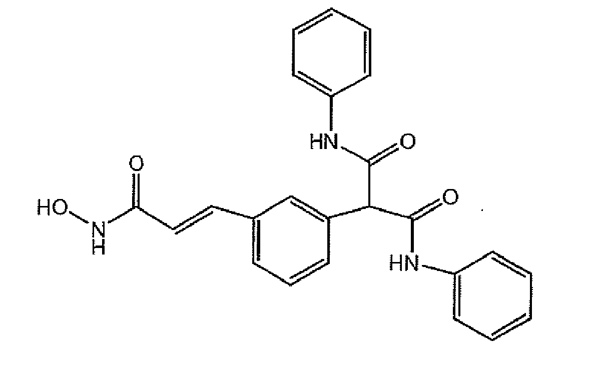 or a pharmaceutically acceptable salt thereof.
72. The method of Clause 59, wherein the histone deacetylase inhibitor compound is represented by structural Formula XXI: 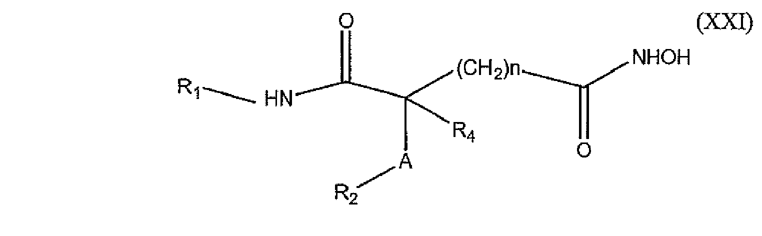 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine, R₄ is hydrogen, a halogen, a phenyl or a cycloalkyl moiety and n is an integer from 3 to 10 or a pharmaceutically acceptable salt thereof.
73. The method of Clause 72, wherein the histone deacetylase inhibitor compound has the structure: 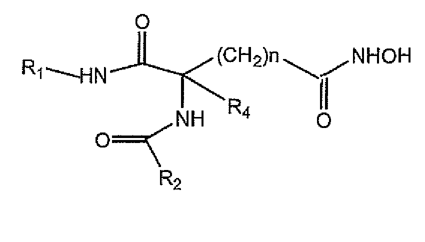 or a pharmaceutically acceptable salt thereof.
74. The method of Clause 72, wherein the histone deacetylase inhibitor compound has the structure: 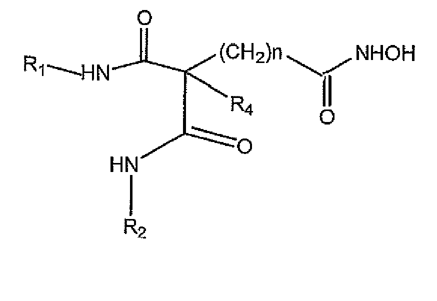 or a pharmaceutically acceptable salt thereof.
75. The method of Clause 59, wherein the histone deacetylase inhibitor compound is represented by structural Formula XVIII: 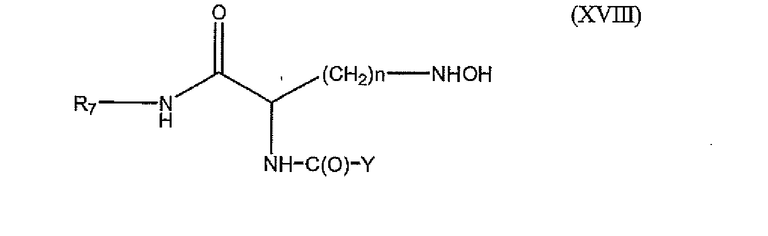 wherein R₇ is selected from substituted or unsubstituted aryl (e.g., phenyl or naphthyl), pyridineamino, 9-purine-60amine, thiazoleamino, aryloxy, arylalkyloxy or pyridine and n is an integer from 3 to 10 and Y is selected from 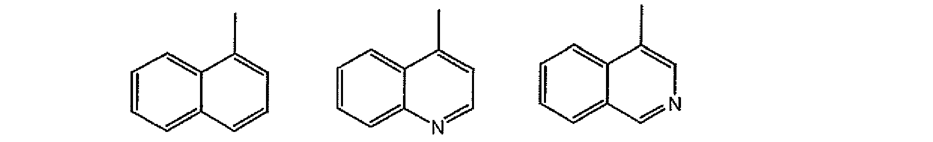 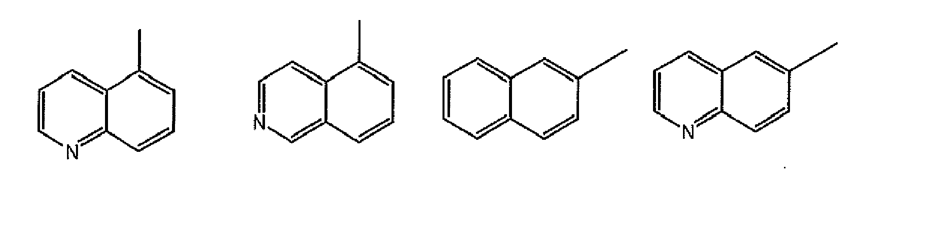 or a pharmaceutically acceptable salt thereof.
76. The method of Clause 59, wherein the histone deacetylase inhibitor compound is represented by structural Formula XIX: 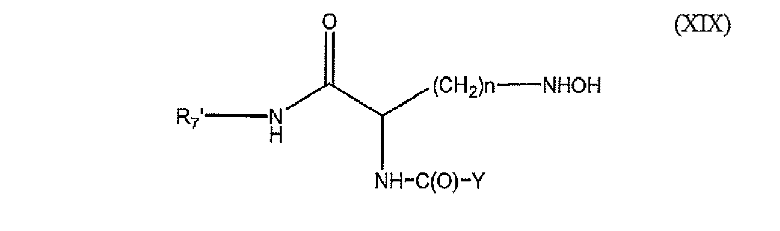 wherein n is an integer from 3 to 10, Y is selected from 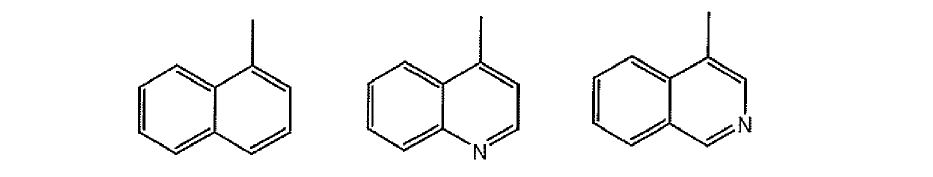 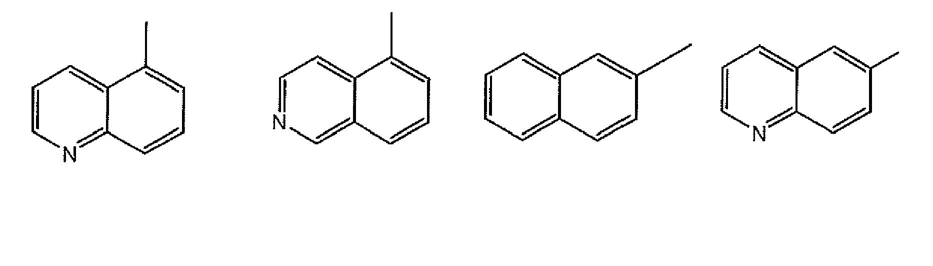 R₇' is selected from 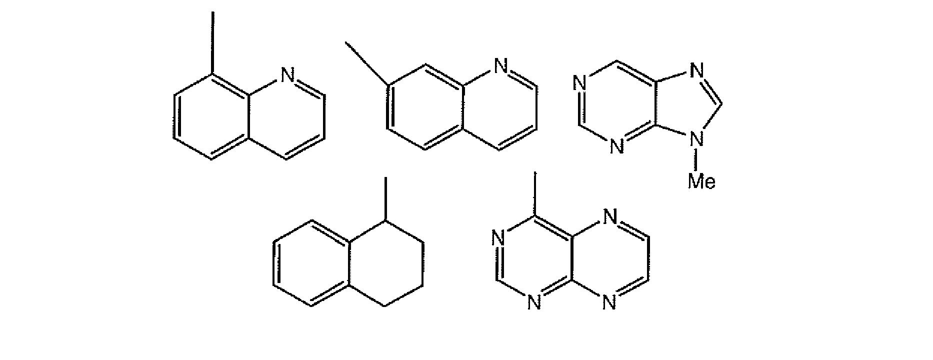 or a pharmaceutically acceptable salt thereof.
77. The method of Clause 59, wherein the histone deacetylase inhibiting compound is represented by structural Formula XX: 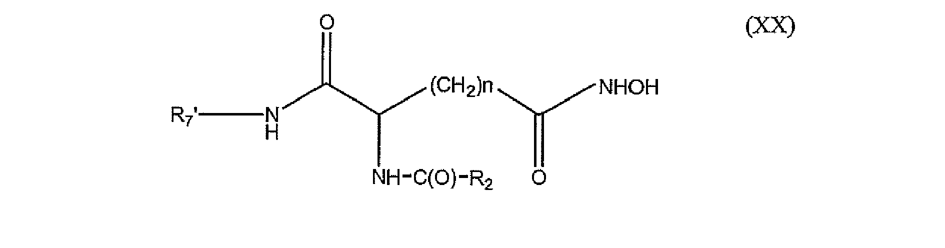 wherein R₂ is selected from substituted or unsubstituted aryl, substituted or unsubstituted naphtha, pyridineamino, 9-purine-6-amine, thiazoleamino, substituted or unsubstituted aryloxy, substituted or unsubstituted arylalkyloxy or pyridine and n is an integer from 3 to 10 and R₇' is selected from 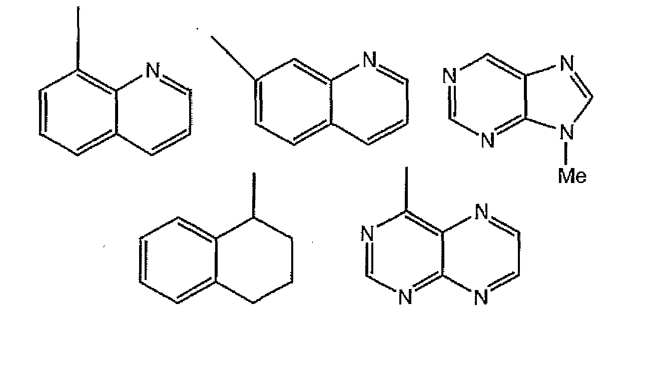 or a pharmaceutically acceptable salt thereof.
78. The method of Clause 59, wherein the histone deacetylase inhibitor compound has the formula: 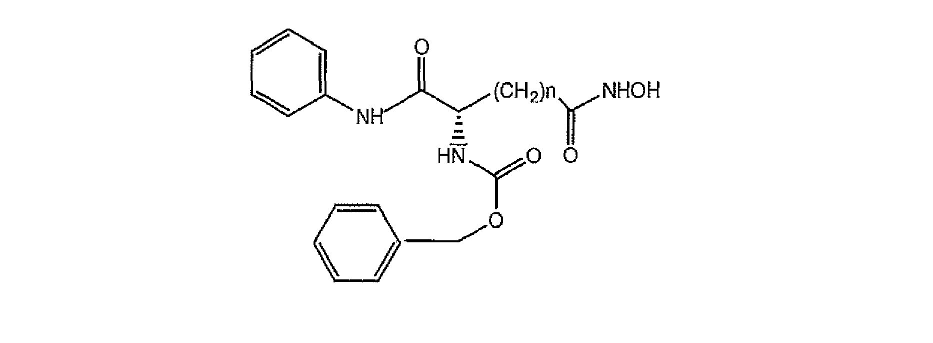 wherein n is an integer from 3 to 10 or a pharmaceuticaly acceptable salt thereof.
79. The method of Clause 59, wherein the histone deacetylase inhibiting compound has the formula: 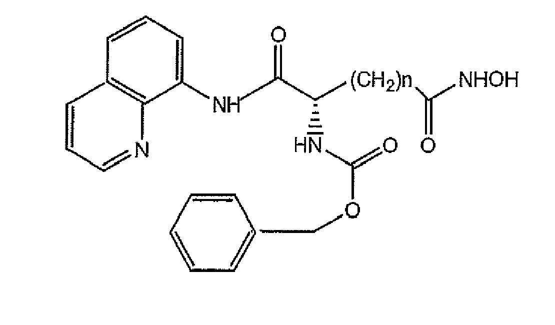 wherein n is an integer from 3 to 10 or a pharmaceutically acceptable salt thereof.
80. The method of Clause 59, wherein the histone deacetylase inhibiting compound has the formula: 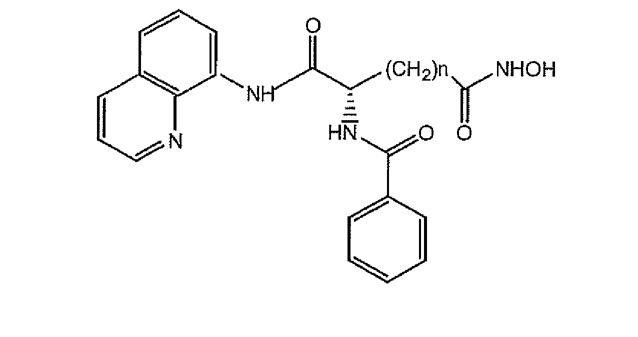 wherein n is an integer from 3 to 10 or a pharmaceutically acceptable salt thereof.
81. The method of Clause 59, wherein the histone deacetylase inhibiting compound has the formula: 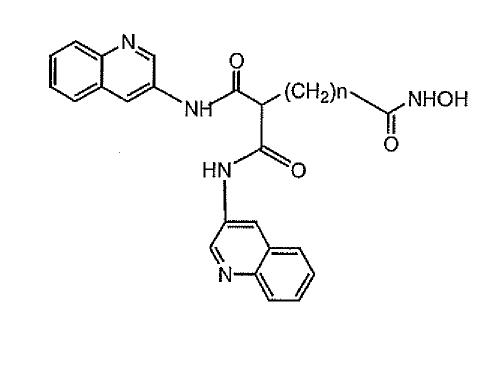 wherein n is an integer from 3 to 10 or a pharmaceutically acceptable salt thereof.
82. The method of Clause 59, wherein the histone deacetylase inhibiting compound has the formula: 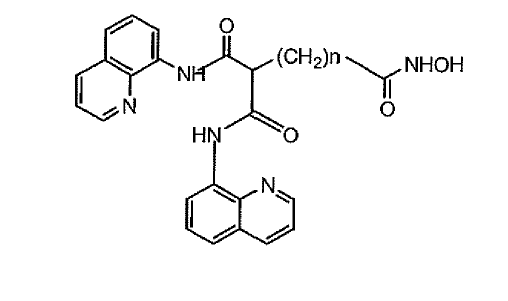 wherein n is an integer from 3 to 10 or a pharmaceutically acceptable salt thereof.
83. The method of Clause 59, wherein the histone deacetylase inhibiting compound has the formula: 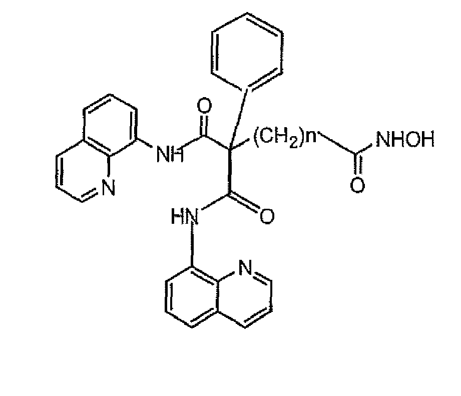 wherein n is an integer from 3 to 10 or a pharmaceutically acceptable salt thereof.
84. The method of Clause 59, wherein the histone deacetylase inhibiting compound has the formula: 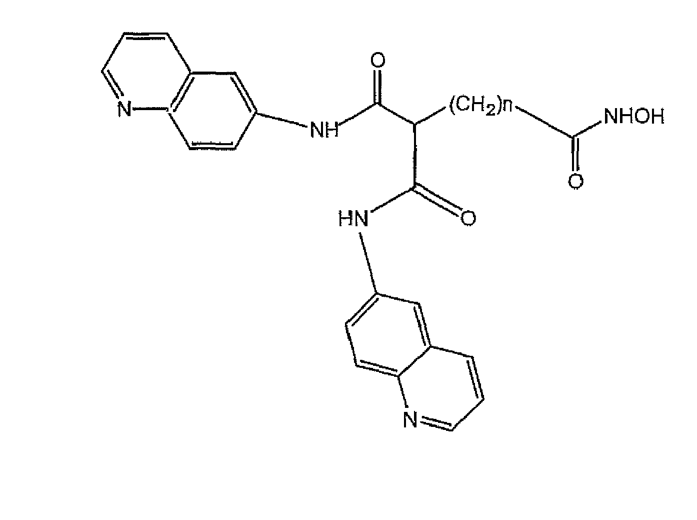 wherein n is an integer from 3 to 10 or a pharmaceutically acceptable salt thereof.
85. The method of Clause 59, wherein the histone deacetylase inhibiting compound has the formula: 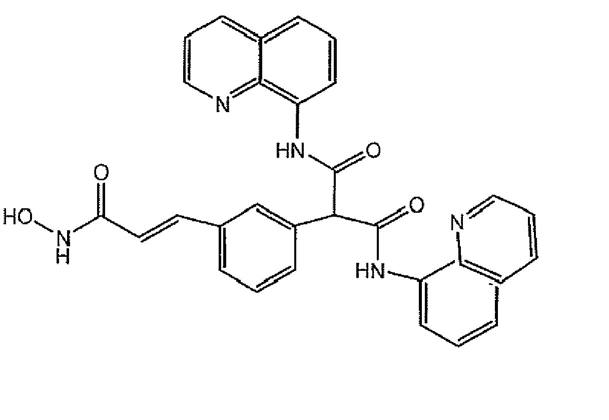 or a pharmaceutically acceptable salt thereof.

## Claims

1. A composition comprising suberoylanilide hydroxamic acid (SAHA) represented by the following structure: 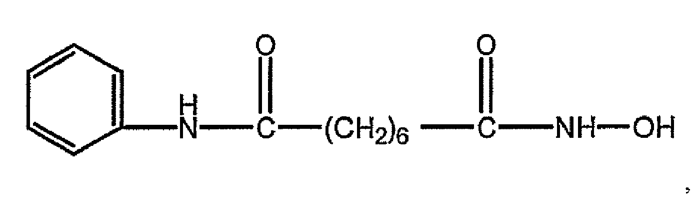 or a pharmaceutically acceptable salt or hydrate thereof, and a pharmaceutically acceptable carrier or diluent, for use in the treatment of brain cancer in a mammal, wherein the composition is formulated for oral administration.

2. A composition for use according to Claim 1, wherein SAHA is the active ingredient in the composition.

3. A composition for use according to Claim 1, wherein the mammal is a human.

4. A composition for use according to Claim 1, wherein the brain cancer is glioma.

5. A composition for use according to Claim 4, wherein the brain cancer is glioblastoma multiforme.

6. A composition for use according to Claim 1, wherein the composition is administered in combination with a standard therapy agent for the disease being treated.
